# EUROPEAN PATENT APPLICATION

(11) **EP 1 362 858 A1**
(43) Date of publication of application: **19.11.2003**
(21) Application number: 02010869.2
(22) Date of filing: 15.05.2002
(51) Int. Cl.: C07D 487/04, A61K 31/519, A61P 9/00, A61P 15/00

(54) **Pyrrolopyrimidinone derivates, process of preparation and use**

(71) Applicant: SK Chemicals Co., Ltd., Suwon, Kyungki-do, 440-301 (KR); In2gen Co., Ltd., Chongno-gu, Seoul 110-799 (KR)
(72) Inventor: Kim, Dae-Kee, Seoul 156-060 (KR); Lee, Ju Young, 741-105 Bidan-maeul best-town, Kyoungki-do, 440-330 (KR); Im, Guang-Jin, 405-902, Hyundai 2 Apt, Kyoungki-do, 425-172 (KR); Choi, Jin-Young, Kyungki-do 440-320 (KR); Kim, Jae-Sun, 102-117 Miyoung Apt., Kyoungki-do, 441-112 (KR); Ryu, Je-Ho,, Seoul, 151-837 (KR); Jung, Jae Yoon, 404 World-villa 2, Kyoungki-do, 472-862 (KR); Lee, JunWon, 638-1404 Sejong Apt., Kyoungki-do, 435-045 (KR); KIm, Tae Kon, 108-211 Woncheon Jukong Apt., Kyoungki-do, 442-757 (KR); Seo, Jung Woo, Kwanack-ku, Seoul, 151-055 (KR); Han, Hye Young, Kwanack-ku, Seoul, 151-821 (KR); Kim, Taek-Soo, Kyoungki-do, 440-817 (KR); Jung, Inho, Kyoungki-do, 440-301 (KR)
(74) Representative: Behnisch, Werner, Dr.

(57) **Abstract**

The invention relates to a series of pyrrolopyrimidinone derivatives, processes for their preparation, intermediates in their preparation, their use as therapeutic agents, and pharmaceutical compositions containing them.

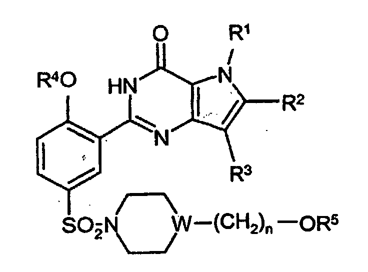

## Description

### BACKGROUND OF THE INVENTION

### Field of the Invention

The invention relates to a series of pyrrolopyrimidinone derivatives of the formula (**1**), processes for their preparation, intermediates in their preparation, their use as therapeutic agents, and pharmaceutical compositions containing them, 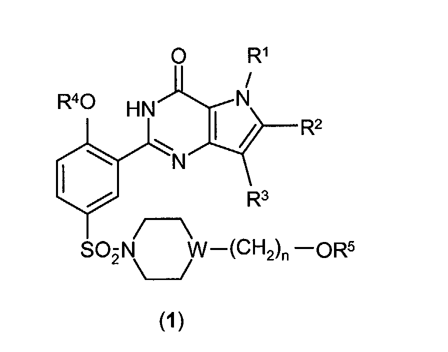 wherein
R¹ is H; or C₁-C₃ alkyl optionally substituted with one or more fluoro atoms;
R² is H; a halogen atom; C₁-C₆ alkyl optionally substituted with OH, or one or more fluoro atoms;
R³ is H; C₁-C₆ alkyl optionally substituted with OH, C₁-C₃ alkoxy, or one or more fluoro atoms; C₃-C₆ cycloalkyl; C₂-C₆ alkenyl; or C₂-C₆ alkynyl;
R⁴ is C₁-C₆ alkyl optionally substituted with one or more fluoro atoms; C₂-C₆ alkenyl; C₂-C₆ alkynyl; or C₃-C₆ cycloalkyl;
W is N; or CH;
n is an integer of from 1 to 6;
R⁵ is COR⁶; COOR⁶; or COCH(R⁷)NH₂;
R⁶ is phenyl or aromatic heterocyclic group wherein said group is optionally substituted with 1 to 4 substituents selected from the group consisting of halogen atom, C₁-C₄ alkyl, C₁-C₄ alkoxy, OH, NH₂, NO₂, CN, COOH, C₁-C₄ aminoalkyl, C₁-C₄ alkylamino, C₁-C₄ acyl, C₁-C₄ acylamino, C₁-C₄ alkylthio, C₁-C₄ perfluoroalkyl, C₁-C₄ perfluoroalkoxy, C₁-C₄ alkoxycarbonyl; C₁-C₁₆ alkyl, C₂-C₁₆ alkenyl or C₂-C₁₆ alkynyl wherein said group is optionally substituted with one or more halogen atoms, C₁-C₄ alkoxy, OH, NH₂, NO₂, CN, COOH, C₁-C₄ aminoalkyl, C₁-C₄ alkylamino, C₁-C₄ acyl, C₁-C₄ acylamino, C₁-C₄ alkylthio, C₁-C₄ perfluoroalkyl, C₁-C₄ perfluoroalkoxy, C₁-C₄ alkoxycarbonyl or with phenyl or aromatic heterocyclic group wherein said group is optionally substituted with 1 to 4 substituents selected from the group consisting of halogen atom, C₁-C₄ alkyl, C₁-C₄ alkoxy, OH, NH₂, NO₂, CN, COOH, C₁-C₄ aminoalkyl, C₁-C₄ alkylamino, C₁-C₄ acyl, C₁-C₄ acylamino, C₁-C₄ alkylthio, C₁-C₄ perfluoroalkyl, C₁-C₄ perfluoroalkoxy and C₁-C₄ alkoxycarbonyl;
R⁷ represents the residue of any naturally occurring amino acid.

### Description of the Prior Art

European patent applications EP-A-0463756 and EP-A-0526004 disclose certain pyrazolo[4,3-*d*]pyrimidin-7-ones as cGMP PDE inhibitors, useful in the treatment of cardiovascular disorders such as angina, hypertension and heart failure. International application WO 94/28902 discloses their use for the treatment of impotence.

The present inventors have recently disclosed a series of pyrazolo[4,3-*d*]pyrimidin-7-one derivatives (Appln. No. KR 98-60436 and KR 99-7580) and a series of pyrrolo[3,2-*d*]pyrimidin-4-one derivatives (PCT Appln. No. KR01/00227) as PDE V inhibitors. Herein a new series of pyrrolo[3,2-*d*]pyrimidin-4-one derivatives are prepared as PDE V inhibitors. However, none of the compounds of this invention are specifically disclosed.

### SUMMARY OF THE INVENTION

The compounds (1) of this invention are potent and selective inhibitors of cyclic guanosine 3',5'-monophosphate specific phosphodiesterase (cGMP specific PDE; PDE V) having utility in a variety of therapeutic areas where such inhibition is thought to be beneficial, including the treatment of impotence (male erectile dysfunction), sexual dysfunction in female, and various cardiovascular disorders such as angina, hypertension, heart failure and atherosclerosis.

As a consequence of the selective PDE V inhibition exhibited by compounds of this invention, cGMP levels are elevated, which in turn can give rise to beneficial vasodilatory, anti-vasospastic, anti-platelet, anti-neutrophil, natriuretic and diuretic activities as well as potentiation of the effects of endothelium-derived relaxing factor (EDRF), nitrovasodilators, atrial natriuretic factor (ANF), brain natriuretic peptide (BNP), C-type natriuretic peptide (CNP) and endothelium-dependent relaxing agents such as bradykinin, acetylcholine and 5-HT₁.

The compounds of this invention therefore have utility in the treatment of a number of disorders, including impotence, sexual dysfunction in female, stable, unstable and variant (Prinzmetal) angina, hypertension, pulmonary hypertension, congestive heart failure, renal failure, atherosclerosis, conditions of reduced blood vessel patency (e. g. post-percutaneous transluminal coronary angioplasty), peripheral vascular disease, vascular disorders such as Raynaud's disease, inflammatory diseases, stroke, bronchitis, chronic asthma, allergic asthma, allergic rhinitis, glaucoma and diseases characterized by disorders of gut motility (e. g. irritable bowel syndrome).

### Detailed Description of the Invention

Thus, according to a first aspect, this invention provides compounds of the formula (**1**) and pharmaceutically acceptable salts and solvates (e.g. hydrates) thereof, 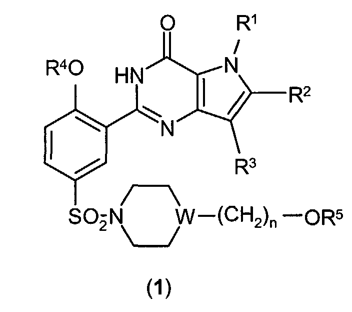 wherein R¹, R², R³, R⁴, W, n and R⁵ are as previously defined.

Throughout this specification, the term aromatic heterocyclic group means a 5-6 membered aromatic ring containing one or more atoms selected from oxygen, sulfur and nitrogen atoms on the ring, said ring being optionally condensed with a carbon ring or other heterocyclic ring.

Examples include pyrrole, indole, carbazole, imidazole, pyrazole, benzimidazole, pyridine, naphthyridine, furopyridine, thienopyridine, pyrrolopyridine, oxazolopyridine, imidazolopyridine, thiazolopyridine, quinoline, isoquinoline, acridine, phenanthridine, pyridazine, pyrimidine, pyrazine, cinnoline, phthaladine, quinazoline, naphthylidine, quinoxaline, isoxazole, benzisoxazole, oxazole, benzoxazole, benzoxadiazole, isothiazole, benzisothiazole, thiazole, benzthiazole, benzthiadiazole, furan, benzofuran, thiophen, benzothiophen and the like.

As used herein, "1 to 4 carbons" means a carbon number per a single substituent; for example, for dialkyl substitution it means 2 to 8 carbons.

A halogen may be fluorine, chlorine, bromine or iodine.

A C₁-C₄ alkyl includes methyl, ethyl, *n*-propyl, isopropyl, *n*-butyl, isobutyl, *sec*-butyl and *tert*-butyl.

A C₁-C₄ alkoxy includes methoxy, ethoxy, *n*-propoxy, isopropoxy, allyloxy, *n*-butoxy, isobutoxy, *sec*-butoxy, *tert*-butoxy and the like.

A C₁-C₄ aminoalkyl includes aminomethyl, 2-aminoethyl, 3-aminopropyl, 4-aminobutyl and the like.

A C₁-C₄ alkylamino includes *N*-methylamino, *N,N*-dimethylamino, *N,N*-diethylamino, *N*-methyl-*N*-ethylamino, *N,N*-diisopropylamino and the like.

A C₁-C₄ acyl includes acetyl, propanoyl, butanoyl and the like.

A C₁-C₄ acylamino includes acetylamino, propanoylamino, butanoylamino and the like.

A C₁-C₄ alkylthio includes methylthio, ethylthio, *n*-propylthio and the like.

A C₁-C₄ perfluoroalkyl includes trifluoromethyl, pentafluoroethyl and the like.

A C₁-C₄ perfluoroalkoxy includes trifluoromethoxy, pentafluoroethoxy and the like.

A C₁-C₄ alkoxycarbonyl includes methoxycarbonyl, ethoxycarbonyl and the like.

Compounds of the formula (**1**) may contain one or more asymmetric centers and thus can exist as enantiomers or diastereomers. It is to be understood that the invention includes both mixtures and separate individual isomers of compounds of the formula **(1)**. Furthermore certain compounds of the formula **(1)** which contain alkenyl groups may exist as cis- or trans-isomers. In each instance, the invention includes both mixtures and separate individual isomers.

Compounds of the formula **(1)** may also exist in tautomeric forms and the invention includes both mixtures and separate individual tautomers thereof.

Also included in the invention are radiolabelled derivatives of compounds of formula (1) which are suitable for biological studies.

Compounds of the formula **(1)** wherein one or more basic nitrogen atoms are present may form pharmaceutically acceptable salts with acids such as hydrochloric, hydrobromic, sulfuric, phosphoric, methanesulfonic, acetic, citric, fumaric, lactic, maleic, succinic and tartaric acids.

Compounds of the formula **(1)** may form pharmaceutically acceptable salts with metal ions, such as alkali metals for example sodium and potassium, or with an ammonium ion.

A preferred group of compounds of the formula **(1)** is that
wherein
R¹ is H; methyl; or ethyl;
R² is H; methyl; or a halogen atom;
R³ is C₁-C₄ alkyl optionally substituted with one or more fluoro atoms;
R⁴ is ethyl; *n*-propyl; or allyl;
W is N;
n is an integer of from 1 to 6;
R⁵ is COR⁶; or COOR⁶;
R⁶ is C₁-C₄ alkyl.

A particularly preferred group of compounds of the formula **(1)** is that
wherein
R¹ is methyl; or ethyl;
R² is H;
R³ is ethyl; 2-fluoroethyl; *n*-propyl; or 3-fluoropropyl;
R⁴ is ethyl; or *n*-propyl;
W is N;
n is an integer of from 2 to 4;
R⁵ is COR⁶; or COOR⁶;
R⁶ is C₁-C₄ alkyl.

Especially preferred individual compounds of the invention include:
2- {5-[4-(2-Acetoxyethyl)piperazin-1-ylsulfonyl]-2-*n*-propoxyphenyl}-5-ethyl-7-*n*-propyl-3,5-dihydro-4*H*-pyrrolo[3,2-*d*]pyrimidin-4-one;
5-Ethyl-2- {5-[4-(2-propionyloxyethyl)piperazin-1-ylsulfonyl]-2-*n*-propoxyphenyl}-7-*n*-propyl-3,5-dihydro-4*H*-pyrrolo[3,2-*d*]pyrimidin-4-one;
2- {5-[4-(2-Butyryloxyethyl)piperazin-1-ylsulfonyl]-2-*n*-propoxyphenyl}-5-ethyl-7-*n*-propyl-3,5-dihydro-4*H*-pyrrolo[3,2-*d*]pyrimidin-4-one;
5-Ethyl-2-{5-[4-(2-isobutyryloxyethyl)piperazin-1-ylsulfonyl]-2-*n*-propoxyphenyl}-7-*n*-propyl-3,5-dihydro-4*H*-pyrrolo[3,2-*d*]pyrimidin-4-one;
5-Ethyl-2- {5-[4-(2-methoxycarbonyloxyethyl)piperazin-1-ylsulfonyl]-2-*n*-propoxyphenyl}-7-*n*-propyl-3,5-dihydro-4*H*-pyrrolo[3,2-*d*]pyrimidin-4-one;
2- {5-[4-(2-Ethoxycarbonyloxyethyl)piperazin-1-ylsulfonyl]-2-*n*-propoxyphenyl}-5-ethyl-7-*n*-propyl-3,5-dihydro-4*H*-pyrrolo[3,2-*d*]pyrimidin-4-one;
5-Ethyl-2- {5-[4-(2-*n*-propoxycarbonyloxyethyl)piperazin-1-ylsulfonyl]-2-*n* propoxyphenyl}-7-*n*-propyl-3,5-dihydro-4*H*-pyrrolo[3,2-*d*]pyrimidin-4-one;
5-Ethyl-2-{5-[4-(2-isopropoxycarbonyloxyethyl)piperazin-1-ylsulfonyl]-2-*n*-propoxyphenyl}-7-*n*-propyl-3,5-dihydro-4*H*-pyrrolo[3,2-*d*]pyrimidin-4-one;
2-{5-[4-(2-*n*-Butoxycarbonyloxyethyl)piperazin-1-ylsulfonyl]-2-*n*-propoxyphenyl}-5-ethyl-7-*n*-propyl-3,5-dihydro-4*H*-pyrrolo[3,2-*d*]pyrimidin-4-one;
2-{5-[4-(2-Acetoxyethyl)piperazin-1-ylsulfonyl]-2-*n*-propoxyphenyl}-5-ethyl-7-(3-fluoropropyl)-3,5-dihydro-4*H*-pyrrolo[3,2-*d*]pyrimidin-4-one;
5-Ethyl-7-(3-fluoropropyl)-2-{5-[4-(2-propionyloxyethyl)piperazin-1-ylsulfonyl]-2-*n*-propoxyphenyl}-3,5-dihydro-4*H*-pyrrolo[3,2-*d*]pyrimidin-4-one;
2-{5-[4-(2-Butyryloxyethyl)piperazin-1-ylsulfonyl]-2-*n*-propoxyphenyl}-5-ethyl-7-(3-fluoropropyl)-3,5-dihydro-4*H*-pyrrolo[3,2-*d*]pyrimidin-4-one;
5-Ethyl-7-(3-fluoropropyl)-2-{5-[4-(2-isobutyryloxyethyl)piperazin-1-ylsulfonyl]-2-*n*-propoxyphenyl}-3,5-dihydro-4*H*-pyrrolo[3,2-*d*]pyrimidin-4-one;
5-Ethyl-7-(3-fluoropropyl)-2-{5-[4-(2-methoxycarbonyloxyethyl)piperazin-1-ylsulfonyl]-2-*n*-propoxyphenyl}-3,5-dihydro-4*H*-pyrrolo[3,2-*d*]pyrimidin-4-one;
2-{5-[4-(2-Ethoxycarbonyloxyethyl)piperazin-1-ylsulfonyl]-2-*n*-propoxyphenyl}-5-ethyl-7-(3-fluoropropyl)-3,5-dihydro-4*H*-pyrrolo[3,2-*d*]pyrimidin-4-one;
5-Ethyl-7-(3-fluoropropyl)-2-{5-[4-(2-*n*-propoxycarbonyloxyethyl)piperazin-1-ylsulfonyl]-2-*n*-propoxyphenyl}-3,5-dihydro-4*H*-pyrrolo[3,2-*d*]pyrimidin-4-one;
5-Ethyl-7-(3-fluoropropyl)-2-{5-[4-(2-isopropoxycarbonyloxyethyl)piperazin-1-ylsulfonyl]-2-*n*-propoxyphenyl}-3,5-dihydro-4*H*-pyrrolo[3,2-*d*]pyrimidin-4-one;
2-{5-[4-(2-*n*-Butoxycarbonyloxyethyl)piperazin-1-ylsulfonyl]-2-*n*-propoxyphenyl}-5-ethyl-7-(3-fluoropropyl)-3,5-dihydro-4*H*-pyrrolo[3,2-*d*]pyrimidin-4-one;
and physiologically acceptable salts and solvates (e. g. hydrates) thereof.

In another aspect, this invention provides processes for the preparation of compounds of the formula **(1)** or pharmaceutically acceptable salts thereof.

Compounds of the general formula **(1)** may be prepared by the reaction of compounds of the general formula **(2)** which are disclosed in the PCT application (KR01/00227): 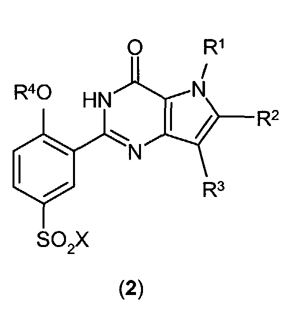 (wherein R¹, R², R³ and R⁴ are as previously defined in the general formula **(1)**, and X represents a halogen atom, preferably a chlorine atom) with a compound of the general formula **(3)**: 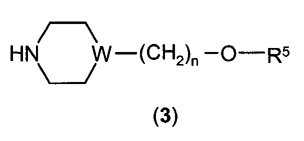 wherein W, n and R⁵ are as previously defined in the general formula **(1)**. The coupling reaction is generally carried out at from 0 °C to room temperature for 1-24 hours in a suitable solvent such as a C₁-C₃ alkanol, dichloromethane, *N,N*-dimethylformamide (DMF) or water using an excess amount of **(3)** or in the presence of an organic tertiary amine such as triethylamine to scavenge the acid by-product.

Compounds of the general formula **(1)** may be also prepared by the esterification reaction of compounds of the general formula **(4)** which are disclosed in the PCT application (KR01/00227): 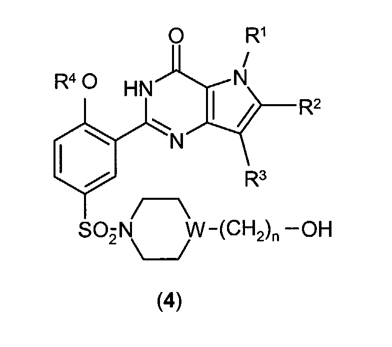 (wherein R¹, R², R³, R⁴, W and n are as previously defined in the general formula (**1**)) with a compound of the general formula (**5**), (**6**) or **(7)**: 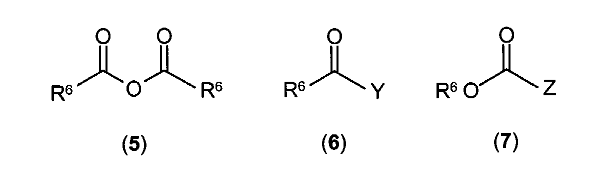 wherein R⁶ is as previously defined, and Y represents a hydroxyl group or a halogen atom, preferably a chlorine atom. Z in the general formula (**7**) represents a halogen atom or a phenoxy group optionally substituted with one or more NO₂ groups. The reaction of (**4**) with (**5**), (**6**) (wherein Y represents a halogen atom) or (**7**) is generally carried out at from 0 °C to room temperature for 1-24 hours in a suitable solvent such as dichloromethane or DMF in the presence of an organic tertiary amine such as triethylamine to scavenge the acid by-product, optionally in the presence of a catalyst such as 4-dimethylaminopyridine (DMAP). For convenience, pyridine may be also used as a solvent. Alternatively, compounds of the general formula **(1)** may be obtained by the treatment of **(4)** with **(6)** (wherein Y represents a hydroxyl group) using an appropriate coupling reagent such as 1,3-dicyclohexylcarbodiimide (DCC) or 1-(3-dimethylaminopropyl)-3-ethylcarbodiimide hydrochloride (EDC) in the presence of an excess of 1-hydroxybenzotriazole, optionally in the presence of a catalyst such as DMAP in an inert solvent such as dichloromethane or DMF at from 0 °C to room temperature for 1-24 hours.

Compounds of the general formula **(4)** may be prepared from compounds of the general formula **(8)**: 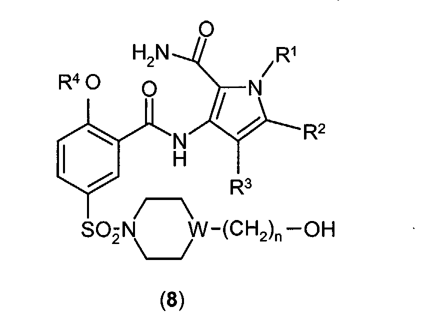 wherein R¹, R², R³, R⁴, W and n are as previously defined in the general formula (**1**). The cyclization reaction is generally carried out by heating at an elevated temperature, for example 50-150 °C, in the presence of an acid or a base in a suitable solvent such as an aqueous C₁-C₄ alkanol, water, a halogenated hydrocarbon or acetonitrile. Thus, for example, the cyclization may be affected by treatment of a compound of the formula **(8)** with a base such as sodium hydroxide, potassium carbonate or potassium *tert*-butoxide in an aqueous alcoholic medium.

Compounds of the general formula **(8)** may be prepared from compounds of the general formula **(9)** and **(10)**: 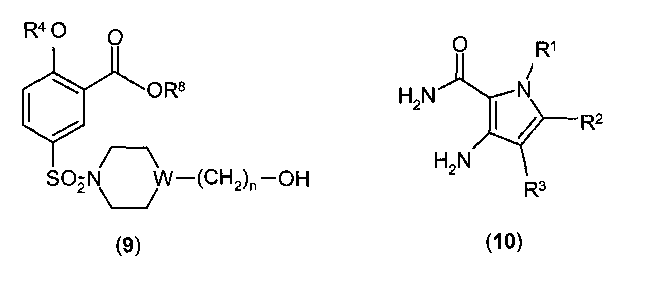 wherein R¹, R², R³, R⁴, W and n are as previously defined in the general formula **(1)**, and R⁸ is H or C₁-C₄ alkyl. The reaction is generally carried out by first converting a carboxylic acid ester of the formula **(9)** to the corresponding carboxylic acid using an excess amount of a well-known reagent in the literature, preferably lithium hydroxide or sodium hydroxide, in a solvent such as tetrahydrofuran-water or ethanol-water at room temperature to reflux temperature. The following coupling reaction of (**9**) (wherein R⁸ is H) with a compound of the formula (**10**) is generally effected by using an excess amount of a well-known reagent in the literature, preferably DCC or EDC, in the presence of an excess amount of 1-hydroxybenzotriazole, optionally in the presence of a catalyst such as DMAP in an inert solvent such as dichloromethane or DMF, at from 0 °C to room temperature for 1-24 hours. For convenience, pyridine may be also used as a solvent.

Compounds of the general formula **(9)** may be prepared from compounds of the general formula **(11)**: 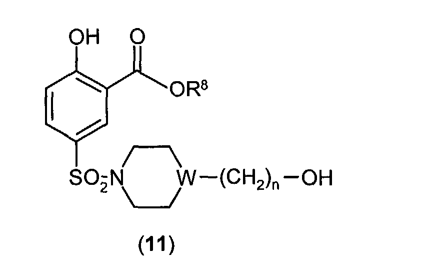 wherein R⁸, W and n are as previously defined in the general formula (**9**). The *O*-alkylation may be effected under a standard condition using an appropriate alkyl halide in the presence of a base such as potassium carbonate in a suitable solvent such as DMF at room temperature to 100°C for 1-24 hours.

Compounds of the general formula **(11)** may be prepared by the reaction of compounds of the general formula **(12)**: 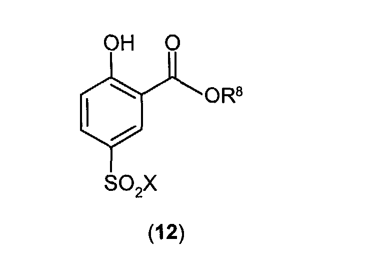 (wherein R⁸ and X are as previously defined in the general formulas (**9**) and (**2**), respectively) with a compound of the general formula **(13)**: 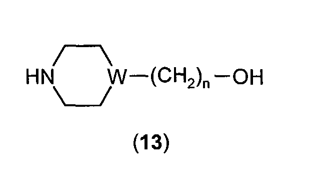 wherein W and n are as previously defined. The coupling reaction is generally carried out at from 0 °C to room temperature for 1-24 hours in a suitable solvent such as a C₁-C₃ alkanol, dichloromethane, DMF or water using an excess amount of **(13)** or in the presence of an organic tertiary amine such as triethylamine or an inorganic base such as potassium carbonate, to scavenge the acid by-product.

Compounds of the general formula **(12)** may be prepared from compounds of the general formula **(14)**: 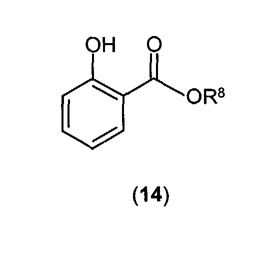 (wherein R⁸ is as previously defined) by using a known method for the introduction of a sulfonyl halide group into an aromatic ring, for example, when halide represents a chlorine atom, by the reaction of chlorosulfonic acid at 0 °C to room temperature for 1-24 hours with or without thionyl chloride.

Compounds of formulas **(3)**, **(5)**, **(6)**, **(7)**, **(10)**, **(13)** and **(14)**, when not commercially available, can be obtained by conventional procedures, in accordance with literature precedent, from readily accessible starting materials using standard reagents and conditions.

The resulting compounds of this invention represented by the formulas **(1)**-**(4)** and **(8)**-**(12)** can be separated and purified by appropriate conventional methods such as column chromatography and recrystallization.

The pharmaceutically acceptable acid addition salts of compounds of the general formula **(1)** which contain a basic center may be prepared in a conventional manner. For example, a solution of the free base is treated with an appropriate acid, either neat or in a suitable solvent, and the resulting salts are isolated either by filtration or by evaporation under vacuum of the reaction solvent.

Compounds of the invention may be administered by any suitable route, for example by oral, buccal, sub-lingual, rectal, vaginal, nasal, topical or parenteral (including intravenous, intramuscular, subcutaneous and intracoronary) administration.

For administration to man in the curative or prophylactic treatment of the disorders identified above, oral, buccal or sub-lingual dosages of a compound of the formula **(1)** will generally be in the range of from 0. 1-400 mg daily for an average adult patient (70 Kg). Thus for a typical adult patient, individual tablets or capsules contain from 0. 05-200 mg of active compound, in a suitable pharmaceutically acceptable vehicle or carrier, for administration in single or multiple doses, once or several times per day. Dosages for parenteral administration will typically be within the range of from 0. 01-100 mg per single dose as required. In practice the physician will determine the actual dosing regimen which will be most suitable for an individual patient, and it will vary with the age, weight and response of the particular patient. The above dosages are exemplary of the average case but there can be individual instances in which higher or lower dosage ranges may be merited, and such are within the scope of this invention.

For human use, a compound of the formula **(1)** can be administered alone, but will generally be administered in admixture with a pharmaceutical carrier selected with regard to the intended route of administration and standard pharmaceutical practice. For example, the compound may be administered orally, buccally or sublingually, in the form of tablets containing excipients such as starch or lactose, or in capsules or ovules either alone or in admixture with excipients, or in the form of elixirs or suspensions containing flavouring or colouring agents. Such liquid preparations may be prepared with pharmaceutically acceptable additives such as suspending agent (e. g. methylcellulose, a semi-synthetic glyceride such as witepsol or mixtures of glycerides such as a mixture of apricot kernel oil and PEG-6 esters or mixtures of PEG-8 and caprylic/capric glycerides). A compound may also be injected parenterally, for example intravenously, intramuscularly, subcutaneously or intracoronarily. For parenteral administration, the compound is best used in the form of a sterile aqueous solution which may contain other substances, for example salts, or monosaccharides such as mannitol or glucose, to make the solution isotonic with blood.

Thus, the invention provides in a further aspect a pharmaceutical composition comprising a compound of the formula **(1)**, or a pharmaceutically acceptable salt thereof, together with a pharmaceutically acceptable diluent or carrier therefor.

The invention also provides a compound of the formula **(1)**, or a pharmaceutically acceptable salt thereof, or a pharmaceutical composition containing either entity, for use in the treatment of impotence, sexual dysfunction in female, stable, unstable and variant (Prinzmetal) angina, hypertension, pulmonary hypertension, congestive heart failure, renal failure, atherosclerosis, conditions of reduced blood vessel patency (e. g. post-percutaneous transluminal coronary angioplasty), peripheral vascular disease, vascular disorders such as Raynaud's disease, inflammatory diseases, stroke, bronchitis, chronic asthma, allergic asthma, allergic rhinitis, glaucoma and diseases characterized by disorders of gut motility (e. g. irritable bowel syndrome).

The invention further provides the use of a compound of the formula **(1)**, or a pharmaceutically acceptable salt thereof, or a pharmaceutical composition containing either entity, for the manufacture of a medicament for the treatment of impotence, sexual dysfunction in female, stable, unstable and variant (Prinzmetal) angina, hypertension, pulmonary hypertension, congestive heart failure, renal failure, atherosclerosis, conditions of reduced blood vessel patency (e. g. post-percutaneous transluminal coronary angioplasty), peripheral vascular disease, vascular disorders such as Raynaud's disease, inflammatory diseases, stroke, bronchitis, chronic asthma, allergic asthma, allergic rhinitis, glaucoma and diseases characterized by disorders of gut motility (e. g. irritable bowel syndrome).

In a further aspect, the invention provides a method of treating or preventing impotence, sexual dysfunction in female, stable, unstable and variant (Prinzmetal) angina, hypertension, pulmonary hypertension, congestive heart failure, renal failure, atherosclerosis, conditions of reduced blood vessel patency (e. g. post-percutaneous transluminal coronary angioplasty), peripheral vascular disease, vascular disorders such as Raynaud's disease, inflammatory diseases, stroke, bronchitis, chronic asthma, allergic asthma, allergic rhinitis, glaucoma and diseases characterized by disorders of gut motility (e. g. irritable bowel syndrome), in a mammal (including a human being), which comprises administering to said mammal a therapeutically effective amount of a compound of formula **(1)**, or a pharmaceutically acceptable salt thereof, or a pharmaceutical composition containing either entity.

### EXAMPLE

The present invention is further illustrated in the following Examples, which should not be taken to limit the scope of the invention.

### Example 1

### Methyl 3-Chlorosulfonyl-6-hydroxybenzoate

To a cooled solution of SOCl₂ (156 g, 1. 31 mol) and ClSO₃H (460 g, 3.94 mol) at 0°C was added slowly methyl salicylate (200 g, 1.31 mol) for 30 minutes, and the mixture was stirred at room temperature for 20 hours. The reaction mixture was poured slowly into the ice (2 kg) and H₂O (3 L) mixture, and the resulting white precipitates were collected by filtration. The filtered solid was washed with H₂O (3 L), air-dried for 2 days and then dried under vacuum at 40°C for 2 days to afford the titled product (232 g, 93%) as a white solid.
mp 76.5-77.5 °C (toluene/hexanes);
IR (neat) 1699 (C=O) cm⁻¹;
¹H NMR (CDCl₃/TMS) δ 3. 90 (s, 3 H, OCH₃), 6. 93 (d, *J*= 8. 7 Hz, 1 H, H-3), 7. 70 (dd, *J*= 8. 7 Hz, 2. 4 Hz, 1 H, H-4), 8. 03 (d, *J*= 2. 4 Hz, 1 H, H-6).

### Example 2

### Methyl 2-Hydroxy-5-[4-(2-hydroxyethyl)piperazin-1-ylsulfonyl]benzoate

To a mixture of 1-(2-hydroxyethyl)piperazine (27 mg, 0. 21 mmol) and K₂CO₃ (33 mg, 0. 24 mmol) in DMF (5 mL) was added methyl 3-chlorosulfonyl-6-hydroxybenzoate (50 mg, 0. 20 mmol), and the mixture was stirred at room temperature for 1 hour. The reaction mixture was washed with H₂O (10 mL), and the aqueous layer was further extracted with 5% MeOH in CH₂Cl₂ (20 mL). The combined organic layer was dried (MgSO₄), filtered, and the filtrate was evaporated to dryness under reduced pressure. The crude residue was purified by MPLC on silica gel (5% MeOH in CH₂Cl₂) to afford the titled compound (59 mg, 86%) as white solid.
mp 152 °C (dec) (CH₂Cl₂/ether);
IR (neat) 1685 (C=O) cm⁻¹;
¹H NMR (CDCl₃/TMS) δ 2. 30 (br s, 1 H, CH₂O*H*), 2. 63 (t, *J* = 5. 4 Hz, 2 H, NC*H*₂CH₂O), 2. 70 (m, 4 H, 2 NCH₂), 3. 12 (m, 4 H, 2 SO₂NCH₂), 3. 64 (t, *J*= 5. 4 Hz, 2 H, NCH₂C*H*₂O), 4. 01 (s, 3 H, OCH₃), 7. 12 (d, *J*= 8. 7 Hz, 1 H, H-3), 7. 81 (dd, *J*= 8. 7 Hz, 2. 4 Hz, 1 H, H-4), 8. 26 (d, *J* = 2. 4 Hz, 1 H, H-6), 11. 26 (br s, 1 H, OH);
MS (FAB) *m*/*z* 345 (MH⁺).

### Example 3

### Methyl 3-[4-(2-Hydroxyethyl)piperazin-1-ylsulfonyl]-6-n-propoxybenzoate

To a mixture of methyl 2-hydroxy-5-(4-(2-hydroxyethyl)piperazin-1-ylsulfonyl)benzoate (800 mg, 2. 32 mmol) and K₂CO₃ (482 mg, 3. 49 mmol) in DMF (5 mL) was added 1-bromopropane (253 µL, 2.79 mmol), and the mixture was stirred at 60°C overnight. The reaction mixture was evaporated to dryness under reduced pressure, washed with H₂O (10 mL), and the aqueous layer was further extracted with CH₂Cl₂ (50 mL x 2). The combined organic layer was dried (MgSO₄), filtered, and the filtrate was evaporated to dryness under reduced pressure. The crude residue was purified by MPLC on silica gel (3% MeOH in CHCl₃) to afford the titled compound (309 mg, 80%) as a white solid.
mp 88-89 °C (EtOAc/hexanes);
IR (neat) 3242 (OH), 1741 (C=O) cm⁻¹;
¹H NMR (CDCl₃/TMS) δ 1. 09 (t, *J* = 7. 5 Hz, 3 H, OCH₂CH₂C*H*₃), 1. 84-1. 95 (m, 2 H, OCH₂C*H*₂CH₃), 2. 23 (br s, 1 H, CH₂O*H*), 2. 54 (t, *J*= 5. 4 Hz, 2 H, NC*H*₂CH₂O), 2. 60 (m, 4 H, 2 NCH₂), 3. 04 (m, 4 H, 2 SO₂NCH₂), 3. 58 (t, *J* = 5. 4 Hz, 2 H, NCH₂C*H*₂O), 3. 91 (s, 3 H, OCH₃), 4. 08 (t, *J*= 6. 6 Hz, 2 H, OC*H*₂CH₂CH₃), 7. 07 (d, *J* = 9. 0 Hz, 1 H, H-3), 7. 82 (dd, *J* = 9. 0 Hz, 2. 4 Hz, 1 H, H-4), 8. 15 (d, *J* = 2. 4 Hz, 1 H, H-6);
MS (FAB) *m*/*z* 387 (MH⁺).

### Example 4

### 2- {5-[4-(2-Acetoxyethyl)piperazin-1-ylsulfonyl]-2-ethoxyphenyl}-5-methyl-7-n-propyl-3,5-dihydro-4H-pyrrolo[3,2-d]pyrimidin-4-one

To a cooled solution of 2-{2-ethoxy-5-[4-(2-hydroxyethyl)piperazin-1-ylsulfonyl]phenyl}-5-methyl-7-*n*-propyl-3,5-dihydro-4*H*-pyrrolo[3,2-*d*]pyrimidin-4-one (100 mg, 0. 20 mmol) and DMAP (12 mg, 0. 10 mmol) in anhydrous pyridine (4 mL) in an ice bath was added slowly acetic anhydride (93 µL, 0. 99 mmol), and the mixture was stirred for 20 minutes. The reaction mixture was evaporated to dryness under reduced pressure, and the resulting residue was diluted with aqueous NaHCO₃ (20 mL), and was extracted with CH₂Cl₂ (30 mL × 2). Combined organic layer was dried (MgSO₄) and filtered, and the filtrate was evaporated to dryness under reduced pressure. The crude product was purified by MPLC on silica gel (1.5% MeOH in CHCl₃) to afford the titled compound (101 mg, 93%) as white solid.
mp 191. 5-192. 5 °C (CHCl₃/ether/hexanes);
IR (neat) 3322 (NH), 1739 (C=O), 1685 (C=O) cm⁻¹;
¹H NMR (CDCl₃/TMS) δ 0. 99 (t, *J*= 7. 2 Hz, 3 H, CH₂CH₂C*H*₃), 1. 64 (t, *J*= 6. 9 Hz, 3 H, OCH₂C*H*₃), 1. 67-1. 79 (m, 2 H, CH₂C*H*₂CH₃), 2. 01 (s, 3 H, O₂CCH₃), 2. 60 (m, 4 H, 2 NCH₂), 2. 62 (t, *J* = 5. 7 Hz, 2 H, NC*H*₂CH₂O), 2. 71 (t, *J* = 7. 5 Hz, 2 H, C*H*₂CH₂CH₃), 3. 11 (m, 4 H, 2 SO₂NCH₂), 4. 08 (s, 3 H, NCH₃), 4. 12 (t, *J*= 5. 7 Hz, 2 H, NCH₂C*H*₂O), 4. 35 (q, *J*= 6. 9 Hz, 2 H, OC*H*₂CH₃), 6. 89 (s, 1 H, H-2), 7. 12 (d, *J*= 8. 7 Hz, 1 H, H-3'), 7. 80 (dd, *J*= 8. 7 Hz, 2. 4 Hz, 1 H, H-4'), 8. 86 (d, *J*= 2. 4 Hz, 1 H, H-6'), 10. 62 (br s, 1 H, NH);
MS (FAB) *m*/*z* 547 (MH⁺).

### Example 5

### 2-{5-[4-(2-Acetoxyethyl)piperazin-1-ylsulfonyl]-2-ethoxyphenyl}-5-methyl-7-n-propyl-3,5-dihydro-4H-pyrrolo[3,2-d]pyrimidin-4-one Hydrogen Sulfate

To a solution of 2-{5-[4-(2-Acetoxyethyl)piperazin-1-ylsulfonyl]-2-ethoxyphenyl}-5-methyl-7-*n*-propyl-3,5-dihydro-4*H*-pyrrolo[3,2-*d*]pyrimidin-4-one (62 mg, 0. 11 mmol) in anhydrous CH₂Cl₂ (4 mL) was added 10% H₂SO₄ in THF (70 µL, 0. 13 mmol) at room temperature under nitrogen atmosphere, and the solution was stirred for about 30 minutes. The reaction mixture was poured slowly into anhydrous ether (20 mL), and the resulting white precipitates were collected by filtration. The filtered solid was dissolved in H₂O (30 mL), filtered through a membrane filter (0. 45 µm), and the filtrate was freeze-dried to afford the titled compound (70 mg, 96%) as a white solid.
IR (neat) 3330 (NH), 1739 (C=O), 1685 (C=O) cm⁻¹;
¹H NMR (DMSO-*d*₆) δ 0. 93 (t, *J*= 7. 2 Hz, 3 H, CH₂CH₂C*H*₃), 1. 37 (t, *J*= 6. 9 Hz, 3 H, OCH₂C*H*₃), 1. 58-1. 70 (m, 2 H, CH₂C*H*₂CH₃), 2. 03 (s, 3 H, O₂CCH₃), 2. 58 (t, *J*= 7. 5 Hz, 2 H, C*H*₂CH₂CH₃), 2. 57-2. 78 (m, 2 H, 2 SO₂NCHₐₓ), 3. 13-4. 18 (m, 8 H, 2 SO₂NCH_{eq}, 2 H⁺NC*H*ₐₓ, 2 H⁺NC*H*_{eq}, and NC*H*₂CH₂O), 3. 99 (s, 3 H, NCH₃), 4. 24 (q, *J* = 6. 9 Hz, 2 H, OC*H*₂CH₃), 4. 19-4. 32 (m, 2 H, NCH₂C*H*₂O), 7. 24 (s, 1 H, H-2), 7. 43 (d, *J* = 8. 7 Hz, 1 H, H-3'), 7. 87 (dd, *J* = 8. 7 Hz, 2. 4 Hz, 1 H, H-4'), 7. 96 (d, *J* = 2. 4 Hz, 1 H, H-6').

### Example 6

### 2-{5-[4-(3-Acetoxypropyl)piperazin-1-ylsulfonyl]-2-ethoxyphenyl}-5-methyl-7-n-propyl-3,5-dihydro-4H-pyrrolo[3,2-d]pyrimidin-4-one

The titled compound was prepared as described in Example 4 by using 2-{2-ethoxy-5-[4-(3-hydroxypropyl)piperazin-1-ylsulfonyl]phenyl}-5-methyl-7-*n*-propyl-3,5-dihydro-4*H*-pyrrolo[3,2-*d*]pyrimidin-4-one in place of 2-{2-ethoxy-5-[4-(2-hydroxyethyl)piperazin-1-ylsulfonyl]phenyl}-5-methyl-7-*n*-propyl-3,5-dihydro-4*H*-pyrrolo[3,2-*d*]pyrimidin-4-one.
yield: 90%
mp 157-158. 5 °C (CH₂Cl₂/EtOAc/ hexanes);
IR (neat) 3334 (NH), 1737 (C=O), 1677 (C=O) cm⁻¹;
¹H NMR (CDCl₃/TMS) δ 0. 99 (t, *J*= 7. 5 Hz, 3 H, CH₂CH₂C*H*₃), 1. 64 (t, *J*= 6. 9 Hz, 3 H, OCH₂C*H*₃), 1. 66-1. 80 (m, 4 H, CH₂C*H*₂CH₃ and CH₂C*H*₂CH₂O), 2. 00 (s, 3 H, O₂CCH₃), 2. 40 (t, *J* = 6. 9 Hz, 2 H, NC*H*₂CH₂CH₂), 2. 52 (m, 4 H, 2 NCH₂), 2. 71 (t, *J* = 7. 5 Hz, 2 H, C*H*₂CH₂CH₃), 3. 09 (m, 4 H, 2 SO₂NCH₂), 4. 04 (t, *J* = 6. 6 Hz, 2 H, CH₂CH₂C*H*₂O), 4. 08 (s, 3 H, NCH₃), 4. 36 (q, *J*= 6. 9 Hz, 2 H, OC*H*₂CH₃), 6. 88 (s, 1 H, H-2), 7. 13 (d, *J*= 8. 7 Hz, 1 H, H-3'), 7. 80 (dd, *J*= 8. 7 Hz, 2. 4 Hz, 1 H, H-4'), 8. 87 (d, *J*= 2. 4 Hz, 1 H, H-6'), 10. 63 (br s, 1 H, NH);
MS (FAB) *m*/*z* 560 (MH⁺).

### Example 7

### 2-{5-[4-(2-Acetoxyethyl)piperazin-1-ylsulfonyl]-2-n-propoxyphenyl}-5-ethyl-7-n-propyl-3,5-dihydro-4H-pyrrolo[3,2-d]pyrimidin-4-one

### (Method A)

A mixture of 2-(5-chlorosulfonyl-2-*n*-propoxyphenyl)-5-ethyl-7-*n*-propyl-3,5-dihydro-4*H*-pyrrolo[3,2-*d*]pyrimidin-4-one (100 mg, 0. 24 mmol) and 1-(2-acetoxyethyl)piperazine dihydrochloride (72 mg, 0. 29 mmol) in EtOH (5 mL) was added Et₃N (204 µL, 1. 46 mmol), and the mixture was stirred overnight at room temperature. The reaction mixture was evaporated to dryness under reduced pressure, and the resulting residue was purified by MPLC on silica gel (2% MeOH in CH₂Cl₂) to afford the titled compound (114 mg, 86%).
mp 139-140 °C (EtOAc/hexanes);
IR (neat) 3323 (NH), 1737 (C=O), 1686 (C=O) cm⁻¹;
¹H NMR (CDCl₃/TMS) δ 1. 00 (t, *J*= 7. 5 Hz, 3 H, CH₂CH₂C*H*₃), 1. 19 (t, *J*= 7. 5 Hz, 3 H, OCH₂CH₂C*H*₃), 1. 48 (t, *J* = 7. 2 Hz, 3 H, NCH₂C*H*₃), 1. 68-1. 81 (m, 2 H, CH₂C*H*₂CH₃), 1. 99-2. 10 (m, 2 H, OCH₂C*H*₂CH₃), 2. 01 (s, 3 H, O₂CCH₃), 2. 60 (m, 4 H, 2 NCH₂), 2. 62 (t, *J* = 5. 7 Hz, 2 H, NC*H*₂CH₂O), 2. 72 (t, *J* = 7. 5 Hz, 2 H, C*H*₂CH₂CH₃), 3. 11 (m, 4 H, 2 SO₂NCH₂), 4. 12 (t, *J* = 5. 7 Hz, 2 H, NCH₂C*H*₂O), 4. 24 (t, *J*= 6. 3 Hz, 2 H, OC*H*₂CH₂CH₃), 4. 45 (q, *J*= 7. 2 Hz, 2 H, NC*H*₂CH₃), 6. 97 (s, 1 H, H-2), 7. 13 (d, *J*= 8. 7 Hz, 1 H, H-3'), 7. 80 (dd, *J*= 8. 7 Hz, 2. 4 Hz, 1 H, H-4'), 8. 89 (d, *J*= 2. 4 Hz, 1 H, H-6'), 10. 69 (br s, 1 H, NH);
MS (FAB) *m*/*z* 574 (MH⁺).

### (Method B)

The titled compound was prepared as described in Example 4 by using 5-ethyl-2-{5-[4-(2-hydroxyethyl)piperazin-1-ylsulfonyl]-2-*n*-propoxyphenyl}-7-*n*-propyl-3,5-dihydro-4*H*-pyrrolo[3,2-*d*]pyrimidin-4-one in place of 2-{2-ethoxy-5-[4-(2-hydroxyethyl)piperazin-1-ylsulfonyl]phenyl}-5-methyl-7-*n*-propyl-3,5-dihydro-4*H*-pyrrolo[3,2-*d*]pyrimidin-4-one.
yield: 72%

### Example 8

### 2-{5-[4-(2-Acetoxyethyl)piperazin-1-ylsulfonyl]-2-n-propoxyphenyl}-5-ethyl-7-n-propyl-3,5-dihydro-4H-pyrrolo[3,2-d]pyrimidin-4-one Hydrogen Sulfate

The titled compound was prepared as described in Example 5 by using 2-{5-[4-(2-acetoxyethyl)piperazin-1-ylsulfonyl]-2-*n*-propoxyphenyl}-5-ethyl-7-*n*-propyl-3,5-dihydro-4*H*-pyrrolo[3,2-*d*]pyrimidin-4-one and 10% H₂SO₄ in EtOH in place of 2-{5-[4-(2-acetoxyethyl)piperazin-1-ylsulfonyl]-2-ethoxyphenyl}-5-methyl-7-*n*-propyl-3,5-dihydro-4*H*-pyrrolo[3,2-*d*]pyrimidin-4-one and 10% H₂SO₄ in THF.
yield: 99%
IR (neat) 1716 (C=O), 1645 (C=O) cm⁻¹;
¹H NMR (DMSO-*d*₆) δ 0. 93 (t, *J*= 7. 2 Hz, 3 H, CH₂CH₂C*H*₃), 0. 97 (t, *J*= 7. 5 Hz, 3 H, OCH₂CH₂C*H*₃), 1. 37 (t, *J* = 7. 2 Hz, 3 H, NCH₂C*H*₃), 1. 58-1. 82 (m, 4 H, CH₂C*H*₂CH₃ and OCH₂C*H*₂CH₃), 2. 04 (s, 3 H, O₂CCH₃), 2. 58 (t, *J* = 7. 5 Hz, 2 H, C*H*₂CH₂CH₃), 2. 62-2. 82 (m, 2 H, 2 SO₂NCHₐₓ), 3. 15-3. 88 (m, 8 H, NC*H*₂CH₂O, 2 SO₂NCH_{eq}, 2 H⁺NC*H*ₐₓ, and 2 H⁺NC*H*_{eq}), 4. 15 (t, *J*= 6. 3 Hz, 2 H, OC*H*₂CH₂CH₃), 4. 29 (br t, *J*= 4. 8 Hz, 2 H, NCH₂C*H*₂O), 4. 38 (q, *J*= 7. 2 Hz, 2 H, NC*H*₂CH₃), 7. 36 (s, 1 H, H-2), 7. 44 (d, *J*= 8. 7 Hz, 1 H, H-3'), 7. 88 (dd, *J*= 8. 7 Hz, 2. 4 Hz, 1 H, H-4'), 7. 98 (d, *J* = 2. 4 Hz, 1 H, H-6').

### Example 9

### 2-{5-[4-(2-Acetoxyethyl)piperazin-1-ylsulfonyl]-2-n-propoxyphenyl}-5-ethyl-7-n-propyl-3,5-dihydro-4H-pyrrolo[3,2-d]pyrimidin-4-one Hydrochloride

The titled compound was prepared as described in Example 5 by using 2-{5-[4-(2-acetoxyethyl)piperazin-1-ylsulfonyl]-2-*n*-propoxyphenyl}-5-ethyl-7-*n*-propyl-3,5-dihydro-4*H*-pyrrolo[3,2-*d*]pyrimidin-4-one and 1M HCl in ether in place of 2-{5-[4-(2-acetoxyethyl)piperazin-1-ylsulfonyl]-2-ethoxyphenyl}-5-methyl-7-*n*-propyl-3,5-dihydro-4*H*-pyrrolo[3,2-*d*]pyrimidin-4-one and 10% H₂SO₄ in THF.
yield: 95%
IR (neat) 1738 (C=O), 1683 (C=O) cm⁻¹;
¹H NMR (DMSO-*d*₆) δ 0. 93 (t, *J*= 7. 2 Hz, 3 H, CH₂CH₂C*H*₃), 0. 96 (t, *J*= 7. 5 Hz, 3 H, OCH₂CH₂C*H*₃), 1. 36 (t, *J* = 7. 2 Hz, 3 H, NCH₂C*H*₃), 1. 57-1. 81 (m, 4 H, CH₂C*H*₂CH₃ and OCH₂C*H*₂CH₃), 2. 04 (s, 3 H, O₂CCH₃), 2. 59 (t, *J* = 7. 5 Hz, 2 H, C*H*₂CH₂CH₃), 2. 82-2. 96 (m, 2 H, 2 SO₂NCHₐₓ), 3. 14-3. 31 (m, 2 H, 2 SO₂NCH_{eq}), 3. 36-3. 47 (m, 2 H, NC*H*₂CH₂O), 3. 49-3. 63 (m, 2 H, 2 H⁺NC*H*ₐₓ), 3. 73-3. 85 (m, 2 H, 2 H⁺NC*H*_{eq}), 4. 15 (t, *J* = 6. 3 Hz, 2 H, OC*H*₂CH₂CH₃), 4. 32-4. 40 (m, 2 H, NCH₂C*H*₂O), 4. 38 (q, *J*= 7. 2 Hz, 2 H, NC*H*₂CH₃), 7. 36 (s, 1 H, H-2), 7. 44 (d, *J*= 8. 7 Hz, 1 H, H-3'), 7. 88 (dd, *J*= 8. 7 Hz, 2. 4 Hz, 1 H, H-4'), 8. 01 (d, *J*= 2. 4 Hz, 1 H, H-6').

### Example 10

### 5-Ethyl-2-{5-[4-(2-propionyloxyethyl)piperazin-1-ylsulfonyl]-2-n-propoxyphenyl}-7-n-propyl-3,5-dihydro-4H-pyrrolo[3,2-d]pyrimidin-4-one

The titled compound was prepared as described in Example 4 by using 5-ethyl-2-{5-[4-(2-hydroxyethyl)piperazin-1-ylsulfonyl]-2-*n*-propoxyphenyl}-7-*n*-propyl-3,5-dihydro-4*H*-pyrrolo[3,2-*d*]pyrimidin-4-one and propionic anhydride in place of 2-{2-ethoxy-5-[4-(2-hydroxyethyl)piperazin-1-ylsulfonyl]phenyl}-5-methyl-7-*n*-propyl-3,5-dihydro-4*H*-pyrrolo[3,2-*d*]pyrimidin-4-one and acetic anhydride.
yield: 96%
mp 126.5-127.5 °C (EtOAc/hexanes);
IR (neat) 3324 (NH), 1733 (C=O), 1686 (C=O) cm⁻¹;
¹H NMR (CDCl₃/TMS) δ 1. 00 (t, *J*= 7. 5 Hz, 3 H, CH₂CH₂C*H*₃), 1. 09 (t, *J*= 7. 5 Hz, 3 H, O₂CCH₂C*H*₃), 1. 19 (t, *J*= 7. 5 Hz, 3 H, OCH₂CH₂C*H*₃), 1. 48 (t, *J*= 7. 2 Hz, 3 H, NCH₂C*H*₃), 1. 68-1. 81 (m, 2 H, CH₂C*H*₂CH₃), 1. 99-2. 10 (m, 2 H, OCH₂C*H*₂CH₃), 2. 28 (q, *J* = 7. 2 Hz, 2 H, O₂CCH₂), 2. 60 (m, 4 H, 2 NCH₂), 2. 62 (t, *J*= 5. 7 Hz, 2 H, NC*H*₂CH₂O), 2. 72 (t, *J* = 7. 5 Hz, 2 H, C*H*₂CH₂CH₃), 3. 10 (m, 4 H, 2 SO₂NCH₂), 4. 13 (t, *J*= 5. 7 Hz, 2 H, NCH₂C*H*₂O), 4. 24 (t, *J*= 6. 3 Hz, 2 H, OC*H*₂CH₂CH₃), 4. 45 (q, *J*= 7. 2 Hz, 2 H, NC*H*₂CH₃), 6. 97 (s, 1 H, H-2), 7. 13 (d, *J*= 8. 7 Hz, 1 H, H-3'), 7. 80 (dd, *J*= 8. 7 Hz, 2. 4 Hz, 1 H, H-4'), 8. 89 (d, *J* = 2. 4 Hz, 1 H, H-6'), 10. 69 (br s, 1 H, NH);
MS (FAB) *m*/*z* 588 (MH⁺).

### Example 11

### 5-Ethyl-2-{5-[4-(2-propionyloxyethyl)piperazin-1-ylsulfonyl]-2-n-propoxyphenyl}-7-n-propyl-3,5-dihydro-4H-pyrrolo[3,2-d]pyrimidin-4-one Hydrogen Sulfate

The titled compound was prepared as described in Example 5 by using 5-ethyl-2-{5-[4-(2-propionyloxyethyl)piperazin-1-ylsulfonyl]-2-*n*-propoxyphenyl}-7-*n*-propyl-3,5-dihydro-4*H*-pyrrolo[3,2-*d*]pyrimidin-4-one and 10% H₂SO₄ in EtOH in place of 2-{5-[4-(2-acetoxyethyl)piperazin-1-ylsulfonyl]-2-ethoxyphenyl}-5-methyl-7-*n*-propyl-3,5-dihydro-4*H*-pyrrolo[3,2-*d*]pyrimidin-4-one and 10% H₂SO₄ in THF.
yield: 97%
IR (neat) 1707 (C=O), 1641 (C=O) cm⁻¹;
¹H NMR (DMSO-*d*₆) δ 0. 93 (t, *J*= 7. 2 Hz, 3 H, CH₂CH₂C*H*₃), 0. 96 (t, *J* = 7. 5 Hz, 3 H, OCH₂CH₂C*H*₃), 1. 02 (t, *J*= 7. 5 Hz, 3 H, O₂CCH₂C*H*₃), 1. 37 (t, *J*= 7. 2 Hz, 3 H, NCH₂C*H*₃), 1. 57-1. 81 (m, 4 H, CH₂C*H*₂CH₃ and OCH₂C*H*₂CH₃), 2. 34 (q, *J*= 7. 5 Hz, 2 H, O₂CC*H*₂CH₃), 2. 58 (t, *J* = 7. 5 Hz, 2 H, C*H*₂CH₂CH₃), 2. 62-2. 78 (m, 2 H, 2 SO₂NCHₐₓ), 3. 18-3. 35 (m, 2 H, 2 SO₂NCH_{eq}), 3. 42-3. 50 (m, 2 H, NC*H*₂CH₂O), 3. 52-3. 86 (m, 4 H, 2 H⁺CC*H*ₐₓ and 2 H⁺NC*H*_{eq}), 4. 15 (t, *J* = 6. 3 Hz, 2 H, OC*H*₂CH₂CH₃), 4. 31 (m, 2 H, NCH₂C*H*₂O), 4. 38 (q, *J*= 7. 2 Hz, 2 H, NC*H*₂CH₃), 7. 38 (s, 1 H, H-2), 7. 46 (d, *J*= 8. 7 Hz, 1 H, H-3'), 7. 90 (dd, *J*= 8. 7 Hz, 2. 4 Hz, 1 H, H-4'), 8. 00 (d, *J*= 2. 4 Hz, 1 H, H-6').

### Example 12

### 2-{5-[4-(2-Butyryloxyethyl)piperazin-1-ylsulfonyl]-2-n-propoxyphenyl}-5-ethyl-7-n-propyl-3,5-dihydro-4H-pyrrolo[3,2-d]pyrimidin-4-one

The titled compound was prepared as described in Example 4 by using 5-ethyl-2-{5-[4-(2-hydroxyethyl)piperazin-1-ylsulfonyl]-2-*n*-propoxyphenyl}-7-*n*-propyl-3,5-dihydro-4*H*-pyrrolo[3,2-*d*]pyrimidin-4-one and butyric anhydride in place of 2-{2-ethoxy-5-[4-(2-hydroxyethyl)piperazin-1-ylsulfonyl]phenyl}-5-methyl-7-*n*-propyl-3,5-dihydro-4*H*-pyrrolo[3,2-*d*]pyrimidin-4-one and acetic anhydride.
yield: 91%
mp 71. 5-73 °C (ether/hexanes);
IR (neat) 3324 (NH), 1737 (C=O), 1673 (C=O) cm⁻¹;
¹H NMR (CDCl₃/TMS) δ 0. 89 (t, *J*= 7. 5 Hz, 3 H, O₂CCH₂CH₂C*H*₃), 1. 00 (t, *J*= 7. 5 Hz, 3 H, CH₂CH₂C*H*₃), 1. 19 (t, *J* = 7. 5 Hz, 3 H, OCH₂CH₂C*H*₃), 1. 48 (t, *J* = 7. 2 Hz, 3 H, NCH₂C*H*₃), 1. 53-1. 66 (m, 2 H, O₂CCH₂C*H*₂CH₃), 1. 68-1. 81 (m, 2 H, CH₂C*H*₂CH₃), 1. 97-2. 12 (m, 2 H, OCH₂C*H*₂CH₃), 2. 24 (t, *J*= 7. 5 Hz, 2 H, O₂CCH₂), 2. 60 (m, 4 H, 2 NCH₂), 2. 61 (t, *J*= 5. 7 Hz, 2 H, NC*H*₂CH₂O), 2. 72 (t, *J* = 7. 5 Hz, 2 H, C*H*₂CH₂CH₃), 3. 10 (m, 4 H, 2 SO₂NCH₂), 4. 13 (t, *J*= 6. 0 Hz, 2 H, NCH₂C*H*₂O), 4. 24 (t, *J*= 6. 6 Hz, 2 H, OC*H*₂CH₂CH₃), 4. 46 (q, *J* = 7. 2 Hz, 2 H, NC*H*₂CH₃), 6. 97 (s, 1 H, H-2), 7. 13 (d, *J* = 8. 7 Hz, 1 H, H-3'), 7. 80 (dd, *J* = 8. 7 Hz, 2. 7 Hz, 1 H, H-4'), 8. 89 (d, *J*= 2. 7 Hz, 1 H, H-6'), 10. 69 (br s, 1 H, NH);
MS (FAB) *m*/*z* 602 (MH⁺).

### Example 13

### 2-{5-[4-(2-Butyryloxyethyl)piperazin-1-ylsulfonyl]-2-n-propoxyphenyl}-5-ethyl-7-n-propyl-3,5-dihydro-4H-pyrrolo[3,2-d]pyrimidin-4-one Hydrogen Sulfate

The titled compound was prepared as described in Example 5 by using 2-{5-[4-(2-butyryloxyethyl)piperazin-1-ylsulfonyl]-2-*n*-propoxyphenyl}-5-ethyl-7-*n*-propyl-3,5-dihydro-4*H*-pyrrolo[3,2-*d*]pyrimidin-4-one and 10% H₂SO₄ in EtOH in place of 2-{5-[4-(2-acetoxyethyl)piperazin-1-ylsulfonyl]-2-ethoxyphenyl}-5-methyl-7-*n*-propyl-3,5-dihydro-4*H*-pyrrolo[3,2-*d*]pyrimidin-4-one and 10% H₂SO₄ in THF.
yield: 82%
IR (neat) 1741 (C=O), 1677 (C=O) cm⁻¹;
¹H NMR (DMSO-*d*₆) δ 0. 88 (t, *J* = 7. 5 Hz, 3 H, O₂CCH₂CH₂C*H*₃), 0. 93 (t, *J* = 7. 2 Hz, 3 H, CH₂CH₂C*H*₃), 0. 97 (t, *J*= 7. 5 Hz, 3 H, OCH₂CH₂C*H*₃), 1. 36 (t, *J*= 7. 2 Hz, 3 H, NCH₂C*H*₃), 1. 48-1. 82 (m, 6 H, O₂CCH₂C*H*₂CH₃, CH₂C*H*₂CH₃, and OCH₂C*H*₂CH₃), 2. 31 (t, *J*= 7. 2 Hz, 2 H, O₂CC*H*₂CH₂CH₃), 2. 58 (t, *J* = 7. 5 Hz, 2 H, C*H*₂CH₂CH₃), 2. 53-2. 78 (m, 2 H, 2 SO₂NCHₐₓ), 3. 14-3. 37 (m, 2 H, 2 SO₂NCH_{eq}), 3. 38-3. 49 (m, 2 H, NC*H*₂CH₂O), 3. 50-3. 66 (m, 2 H, 2 H⁺NC*H*ₐₓ), 3. 67-3. 90 (m, 2 H, 2 H⁺NC*H*_{eq}), 4. 15 (t, *J*= 6. 5 Hz, 2 H, OC*H*₂CH₂CH₃), 4. 30 (m, 2 H, NCH₂C*H*₂O), 4. 38 (q, *J* = 7. 2 Hz, 2 H, NC*H*₂CH₃), 7. 34 (s, 1 H, H-2), 7. 44 (d, *J* = 9. 0 Hz, 1 H, H-3'), 7. 87 (dd, *J*= 9. 0 Hz, 2. 4 Hz, 1 H, H-4'), 7. 98 (d, *J*= 2. 4 Hz, 1 H, H-6'), 9. 37 (br s, 1 H, NH⁺), 11. 78 (br s, 1 H, NH).

### Example 14

### 5-Ethyl-2-{5-[4-(2-isobutyryloxyethyl)piperazin-1-ylsulfonyl]-2-n-propoxyphenyl}-7-n-propyl-3,5-dihydro-4H-pyrrolo[3,2-d]pyrimidin-4-one

The titled compound was prepared as described in Example 4 by using 5-ethyl-2-{5-[4-(2-hydroxyethyl)piperazin-1-ylsulfonyl]-2-*n*-propoxyphenyl}-7-*n*-propyl-3,5-dihydro-4*H*-pyrrolo[3,2-*d*]pyrimidin-4-one and isobutyric anhydride in place of 2-{2-ethoxy-5-[4-(2-hydroxyethyl)piperazin-1-ylsulfonyl]phenyl}-5-methyl-7-*n*-propyl-3,5-dihydro-4*H*-pyrrolo[3,2-*d*]pyrimidin-4-one and acetic anhydride.
yield: 94%
mp 88-89 °C (ether/hexanes);
IR (neat) 3325 (NH), 1731 (C=O), 1686 (C=O) cm⁻¹;
¹H NMR (CDCl₃/TMS) δ 1. 00 (t, *J*= 7. 2 Hz, 3 H, CH₂CH₂C*H*₃), 1. 11 (d, *J*= 6. 9 Hz, 6 H, CH(C*H*₃)₂), 1. 19 (t, *J* = 7. 5 Hz, 3 H, OCH₂CH₂C*H*₃), 1. 48 (t, *J* = 7. 2 Hz, 3 H, NCH₂C*H*₃), 1. 68-1. 80 (m, 2 H, CH₂C*H*₂CH₃), 1. 99-2. 10 (m, 2 H, OCH₂C*H*₂CH₃), 2. 43-2. 56 (m, 1 H, O₂CCH), 2. 61 (m, 4 H, 2 NCH₂), 2. 62 (t, *J* = 5. 7 Hz, 2 H, NC*H*₂CH₂O), 2. 72 (t, *J*= 7. 5 Hz, 2 H, C*H*₂CH₂CH₃), 3. 09 (m, 4 H, 2 SO₂NCH₂), 4. 12 (t, *J*= 5. 7 Hz, 2 H, NCH₂C*H*₂O), 4. 24 (t, *J*= 6. 6 Hz, 2 H, OC*H*₂CH₂CH₃), 4. 45 (q, *J*= 7. 2 Hz, 2 H, NC*H*₂CH₃), 6. 97 (s, 1 H, H-2), 7. 13 (d, *J*= 8. 7 Hz, 1 H, H-3'), 7. 80 (dd, *J*= 8. 7 Hz, 2. 4 Hz, 1 H, H-4'), 8. 89 (d, *J*= 2. 4 Hz, 1 H, H-6'), 10. 69 (br s, 1 H, NH);
MS (FAB) *m*/*z* 602 (MH⁺).

### Example 15

### 5-Ethyl-2-{5-[4-(2-isobutyryloxyethyl)piperazin-1-ylsulfonyl]-2-n-propoxyphenyl}-7-n-propyl-3,5-dihydro-4H-pyrrolo[3,2-d]pyrimidin-4-one Hydrogen Sulfate

The titled compound was prepared as described in Example 5 by using 5-ethyl-2-{5-[4-(2-isobutyryloxyethyl)piperazin-1-ylsulfonyl]-2-*n*-propoxyphenyl}-7-*n*-propyl-3,5-dihydro-4*H*-pyrrolo[3,2-*d*]pyrimidin-4-one and 10% H₂SO₄ in EtOH in place of 2-{5-[4-(2-acetoxyethyl)piperazin-1-ylsulfonyl]-2-ethoxyphenyl}-5-methyl-7-*n*-propyl-3,5-dihydro-4*H*-pyrrolo[3,2-*d*]pyrimidin-4-one and 10% H₂SO₄ in THF.
yield: 99%
IR (neat) 1739 (C=O), 1683 (C=O) cm⁻¹;
¹H NMR (DMSO-*d*₆) δ 0. 93 (t, *J*= 7. 2 Hz, 3 H, CH₂CH₂C*H*₃), 0. 97 (t, *J*= 7. 5 Hz, 3 H, OCH₂CH₂C*H*₃), 1. 09 (d, *J*= 6. 9 Hz, 6 H, CH(C*H*₃)₂), 1. 36 (t, *J*= 7. 2 Hz, 3 H, NCH₂C*H*₃), 1. 58-1. 82 (m, 4 H, CH₂C*H*₂CH₃ and OCH₂C*H*₂CH₃), 2. 53-2. 78 (m, 5 H, O₂CCH, C*H*₂CH₂CH₃, and 2 SO₂NCHₐₓ), 3. 17-3. 38 (m, 2 H, 2 SO₂NCH_{eq}), 3. 42-3. 51 (m, 2 H, NC*H*₂CH₂O), 3. 52-3. 66 (m, 2 H, 2 H⁺NC*H*ₐₓ), 3. 71-3. 88 (m, 2 H, 2 H⁺NC*H*_{eq}), 4. 15 (t, *J*= 6. 3 Hz, 2 H, OC*H*₂CH₂CH₃), 4. 30 (m, 2 H, NCH₂C*H*₂O), 4. 38 (q, *J*= 7. 2 Hz, 2 H, NC*H*₂CH₃), 7. 35 (s, 1 H, H-2), 7. 45 (d, *J*= 8. 7 Hz, 1 H, H-3'), 7. 88 (dd, *J*= 8. 7 Hz, 2. 7 Hz, 1 H, H-4'), 7. 98 (d, *J*= 2. 7 Hz, 1 H, H-6'), 9. 41 (br s, 1 H, NH⁺).

### Example 16

### 2-{5-[4-(2-Benzoyloxyethyl)piperazin-1-ylsulfonyl]-2-n-propoxyphenyl}-5-ethyl-7-n-propyl-3,5-dihydro-4H-pyrrolo[3,2-d]pyrimidin-4-one

The titled compound was prepared as described in Example 4 by using 5-ethyl-2-{5-[4-(2-hydroxyethyl)piperazin-1-ylsulfonyl]-2-*n*-propoxyphenyl}-7-*n*-propyl-3,5-dihydro-4*H*-pyrrolo[3,2-*d*]pyrimidin-4-one and benzoic anhydride in place of 2-{2-ethoxy-5-[4-(2-hydroxyethyl)piperazin-1-ylsulfonyl]phenyl}-5-methyl-7-*n*-propyl-3,5-dihydro-4*H*-pyrrolo[3,2-*d*]pyrimidin-4-one and acetic anhydride.
yield: 99%
mp 78-79 °C (EtOAc/ether);
IR (neat) 3338 (NH), 1722 (C=O), 1659 (C=O) cm⁻¹;
¹H NMR (CDCl₃/TMS) δ 0. 99 (t, *J*= 7. 2 Hz, 3 H, CH₂CH₂C*H*₃), 1. 19 (t, *J*= 7. 5 Hz, 3 H, OCH₂CH₂C*H*₃), 1. 48 (t, *J* = 7. 2 Hz, 3 H, NCH₂C*H*₃), 1. 67-1. 80 (m, 2 H, CH₂C*H*₂CH₃), 1. 98-2. 10 (m, 2 H, OCH₂C*H*₂CH₃), 2. 68 (m, 4 H, 2 NCH₂), 2. 71 (t, *J* = 7. 5 Hz, 2 H, C*H*₂CH₂CH₃), 2. 77 (t, *J*= 5. 7 Hz, 2 H, NC*H*₂CH₂O), 3. 11 (m, 4 H, 2 SO₂NCH₂), 4. 23 (t, *J* = 6. 6 Hz, 2 H, OC*H*₂CH₂CH₃), 4. 38 (t, *J* = 5. 7 Hz, 2 H, NCH₂C*H*₂O), 4. 45 (q, *J*= 7. 2 Hz, 2 H, NC*H*₂CH₃), 6. 96 (s, 1 H, H-2), 7. 13 (d, *J*= 8. 7 Hz, 1 H, H-3'), 7. 38-7. 43 (m, 2 H, 2 Ph-H), 7. 51-7. 56 (m, 1 H, Ph-H), 7. 80 (dd, *J* = 8. 7 Hz, 2. 4 Hz, 1 H, H-4'), 7. 96-7. 98 (m, 2 H, 2 Ph-H), 8. 89 (d, *J*= 2. 4 Hz, 1 H, H-6'), 10. 68 (br s, 1 H, NH);
MS (FAB) *m*/*z* 636 (MH⁺).

### Example 17

### 2-{5-[4-(2-Ethoxycarbonyloxyethyl)piperazin-1-ylsulfonyl]-2-n-propoxyphenyl}-5-ethyl-7-n-propyl-3,5-dihydro-4H-pyrrolo[3,2-d]pyrimidin-4-one

A mixture of 5-ethyl-2-{5-[4-(2-hydroxyethyl)piperazin-1-ylsulfonyl]-2-*n*-propoxyphenyl}-7-*n*-propyl-3,5-dihydro-4*H*-pyrrolo[3,2-*d*]pyrimidin-4-one (300 mg, 0. 56 mmol), ethyl 4-nitrophenycarbonate (131 mg, 0. 62 mmol), and DMAP (14 mg, 0. 11 mmol) in pyridine (10 mL) was stirred overnight at 80-90 °C. The reaction mixture was evaporated to dryness under reduced pressure. The crude residue was purified by MPLC on silica gel (3% MeOH in EtOAc) to afford the titled compound (198 mg, 58%) as a white solid.
mp 135-136 °C (EtOAc/ether);
IR (neat) 3330 (NH), 1744 (C=O), 1688 (C=O) cm⁻¹;
¹H NMR (CDCl₃/TMS) δ 1. 00 (t, *J*= 7. 5 Hz, 3 H, CH₂CH₂C*H*₃), 1. 19 (t, *J*= 7. 5 Hz, 3 H, OCH₂CH₂C*H*₃), 1. 25 (t, *J*= 7. 2 Hz, 3 H, OCO₂CH₂C*H*₃), 1. 48 (t, *J*= 7. 2 Hz, 3 H, NCH₂C*H*₃), 1. 68-1. 81 (m, 2 H, CH₂C*H*₂CH₃), 1. 99-2. 11 (m, 2 H, OCH₂C*H*₂CH₃), 2. 61 (m, 4 H, 2 NCH₂), 2. 65 (t, *J*= 5. 7 Hz, 2 H, NC*H*₂CH₂O), 2. 72 (t, *J*= 7. 5 Hz, 2 H, C*H*₂CH₂CH₃), 3. 10 (m, 4 H, 2 SO₂NCH₂), 4. 13 (q, *J* = 7. 2 Hz, 2 H, OCO₂C*H*₂CH₃), 4. 17 (t, *J* = 5. 7 Hz, 2 H, NCH₂C*H*₂O), 4. 25 (t, *J* = 6. 6 Hz, 2 H, OC*H*₂CH₂CH₃), 4. 45 (q, *J*= 7. 2 Hz, 2 H, NC*H*₂CH₃), 6. 97 (s, 1 H, H-2), 7. 13 (d, *J*= 8. 7 Hz, 1 H, H-3'), 7. 80 (dd, *J*= 8. 7 Hz, 2. 4 Hz, 1 H, H-4'), 8. 89 (d, *J*= 2. 4 Hz, 1 H, H-6'), 10. 69 (br s, 1 H, NH);
MS (FAB) *m*/*z* 604 (MH⁺).

### Example 18

### 2-{5-[4-(2-Ethoxycarbonyloxyethyl)piperazin-1-ylsulfonyl]-2-n-propoxyphenyl}-5-ethyl-7-n-propyl-3,5-dihydro-4H-pyrrolo[3,2-d]pyrimidin-4-one Hydrogen Sulfate

The titled compound was prepared as described in Example 5 by using 2-{5-[4-(2-ethoxycarbonyloxyethyl)piperazin-1-ylsulfonyl]-2-*n*-propoxyphenyl}-5-ethyl-7-*n* propyl-3,5-dihydro-4*H*-pyrrolo[3,2-*d*]pyrimidin-4-one and 10% H₂SO₄ in EtOH in place of 2-{5-[4-(2-acetoxyethyl)piperazin-1-ylsulfonyl]-2-ethoxyphenyl}-5-methyl-7-*n*-propyl-3,5-dihydro-4*H*-pyrrolo[3,2-*d*]pyrimidin-4-one and 10% H₂SO₄ in THF.
yield: 97%
IR (neat) 1748 (C=O), 1719 (C=O) cm⁻¹;
¹H NMR (DMSO-*d*₆) δ 0. 92 (t, *J*= 7. 2 Hz, 3 H, CH₂CH₂C*H*₃), 0. 95 (t, *J*= 7. 5 Hz, 3 H, OCH₂CH₂C*H*₃), 1. 21 (t, *J*= 7. 2 Hz, 3 H, OCO₂CH₂C*H*₃), 1. 37 (t, *J*= 7. 2 Hz, 3 H, NCH₂C*H*₃), 1. 57-1. 80 (m, 4 H, CH₂C*H*₂CH₃ and OCH₂C*H*₂CH₃), 2. 58 (t, *J*= 7. 5 Hz, 2 H, C*H*₂CH₂CH₃), 2. 62-2. 81 (m, 2 H, 2 SO₂NCHₐₓ), 3. 17-3. 37 (m, 2 H, 2 SO₂NCH_{eq}), 3. 45-3. 88 (m, 6 H, NC*H*₂CH₂O, 2 H⁺NC*H*ₐₓ and 2 H⁺NC*H*_{eq}), 4. 09-4. 20 (m, 4 H, OCO₂C*H*₂CH₃ and OC*H*₂CH₂CH₃), 4. 33-4. 45 (m, 4 H, NC*H*₂CH₃ and NCH₂C*H*₂O), 7. 42 (s, 1 H, H-2), 7. 47 (d, *J*= 8. 7 Hz, 1 H, H-3'), 7. 92 (d, *J*= 8. 7 Hz, 1 H, H-4'), 8. 02 (s, 1 H, H-6').

### Example 19

### 2-{5-[4-(2-Acetoxyethyl)piperazin-1-ylsulfonyl]-2-n-propoxyphenyl}-5-ethyl-7-(3-fluoropropyl)-3,5-dihydro-4H-pyrrolo[3,2-d]pyrimidin-4-one

The titled compound was prepared as described in Example 4 by using 5-ethyl-7-(3-fluoropropyl)-2-{5-[4-(2-hydroxyethyl)piperazin-1-ylsulfonyl]-2-*n*-propoxyphenyl}-3,5-dihydro-4*H*-pyrrolo[3,2-*d*]pyrimidin-4-one in place of 2-{2-ethoxy-5-[4-(2-hydroxyethyl)piperazin-1-ylsulfonyl]phenyl}-5-methyl-7-*n*-propyl-3,5-dihydro-4*H*-pyrrolo[3,2-d]pyrimidin-4-one.
yield: 80%
mp 134-135 °C (EtOAc/hexanes);
IR (neat) 3319 (NH), 1740 (C=O), 1689 (C=O) cm⁻¹;
¹H NMR (CDCl₃/TMS) δ 1. 20 (t, *J*= 7. 5 Hz, 3 H, CH₂CH₂C*H*₃), 1. 49 (t, *J*= 7. 2 Hz, 3 H, NCH₂C*H*₃), 2. 00-2. 23 (m, 4 H, CH₂C*H*₂CH₃ and CH₂C*H*₂CH₂F), 2. 01 (s, 3 H, O₂CCH₃), 2. 60 (m, 4 H, 2 NCH₂), 2. 62 (t, *J*= 5. 7 Hz, 2 H, NC*H*₂CH₂O), 2. 87 (t, *J*= 7. 5 Hz, 2 H, C*H*₂CH₂CH₂F), 3. 10 (m, 4 H, 2 SO₂NCH₂), 4. 12 (t, *J* = 5. 7 Hz, 2 H, NCH₂C*H*₂O), 4. 25 (t, *J* = 6. 3 Hz, 2 H, C*H*₂CH₂CH₃), 4. 46 (q, *J* = 7. 2 Hz, 2 H, NC*H*₂CH₃), 4. 52 (dt, *J*= 47. 1 Hz, 5. 7 Hz, 2 H, CH₂CH₂C*H*₂F), 7. 00 (s, 1 H, H-2), 7. 14 (d, *J*= 8. 7 Hz, 1 H, H-3'), 7. 81 (dd, *J*= 8. 7 Hz, 2. 4 Hz, 1 H, H-4'), 8. 88 (d, *J*= 2. 4 Hz, 1 H, H-6'), 10. 73 (br s, 1 H, NH);
MS (FAB) *m*/*z* 592 (MH⁺).

### Example 20

### 2-{5-[4-(2-(N-(tert-Butoxycarbonyl)-L-valinyl)oxyethyl)piperazin-1-ylsulfonyl]-2-ethoxyphenyl}-5-methyl-7-n-propyl-3,5-dihydro-4H-pyrrolo[3,2-d]pyrimidin-4-one

To a mixture of 2-{2-ethoxy-5-[4-(2-hydroxyethyl)piperazin-1-ylsulfonyl]phenyl}-5-methyl-7-*n*-propyl-3,5-dihydro-4*H*-pyrrolo[3,2-*d*]pyrimidin-4-one (200 mg, 0. 40 mmol), *N*-(*tert*-butoxycarbonyl)-L-valine (172 mg, 0. 79 mmol), and DMAP (10 mg, 0. 08 mmol) in CH₂Cl₂ (10 mL) was added EDC (114 mg, 0. 59 mmol), and the mixture was stirred for 2 hours at room temperature. The reaction mixture was washed with dilute NaHCO₃ aqueous solution (30 mL), and the aqueous layer was further extracted with CH₂Cl₂ (30 mL). The combined organic layer was dried (MgSO₄), filtered, and the filtrate was evaporated to dryness under reduced pressure. The crude residue was purified by MPLC on silica gel (1.5% MeOH in CHCl₃) to afford the titled compound (258 mg, 92%) as a pale yellow solid.
mp 166. 5 °C (dec) (EtOAc/ether/hexanes);
IR (neat) 3333 (NH), 1722 (C=O), 1677 (C=O) cm⁻¹;
¹H NMR (CDCl₃/TMS) δ 0. 81 (d, *J*= 6. 9 Hz, 3 H, CHC*H*₃), 0. 89 (d, *J*= 6. 9 Hz, 3 H, CHC*H*₃), 1. 00 (t, *J*= 7. 5 Hz, 3 H, CH₂CH₂C*H*₃), 1. 41 (s, 9 H, 3 CH₃), 1. 64 (t, *J*= 6. 9 Hz, 3 H, OCH₂C*H*₃), 1. 66-1. 80 (m, 2 H, CH₂C*H*₂CH₃), 1. 98-2. 11 (m, 1 H, C*H*CH₃), 2. 57 (m, 4 H, 2 NCH₂), 2. 62 (t, *J*= 5. 7 Hz, 2 H, NC*H*₂CH₂O), 2. 71 (t, *J*= 7. 5 Hz, 2 H, C*H*₂CH₂CH₃), 3. 09 (m, 4 H, 2 SO₂NCH₂), 4. 08 (s, 3 H, NCH₃), 4. 15 (t, *J*= 5. 7 Hz, 2 H, NCH₂C*H*₂O), 4. 18-4. 29 (m, 1 H, COCH), 4. 36 (q, *J* = 6. 9 Hz, 2 H, OC*H*₂CH₃), 4. 94 (br d, *J*= 9. 3 Hz, 1 H, NHBoc), 6. 88 (s, 1 H, H-2), 7. 13 (d, *J*= 8. 7 Hz, 1 H, H-3'), 7. 80 (dd, *J*= 8. 7 Hz, 2. 4 Hz, 1 H, H-4'), 8. 87 (d, *J*= 2. 4 Hz, 1 H, H-6'), 10. 64 (br s, 1 H, NH);
MS (FAB) *m*/*z* 704 (MH⁺).

### Example 21

### 2-{2-Ethoxy-5-[4-(2-(L-valinyl)oxyethyl)piperazin-1-ylsulfonyl]phenyl}-5-methyl-7-n-propyl-3,5-dihydro-4H-pyrrolo[3,2-d]pyrimidin-4-one Dihydrochloride

To a cooled solution of 2-{5-[4-(2-(*N*-(*tert*-Butoxycarbonyl)-L-valinyl)oxyethyl)piperazin-1-ylsulfonyl]-2-ethoxyphenyl}-5-methyl-7-*n*-propyl-3,5-dihydro-4*H*-pyrrolo[3,2-*d*]pyrimidin-4-one (150 mg, 0. 21 mmol) in CH₂Cl₂ (5 mL) at 0°C was added slowly CF₃CO₂H (49 µL, 6. 39 mmol), and the mixture was stirred overnight at room temperature. The reaction mixture was evaporated to dryness under vacuum. The crude residue was dissolved in THF (2 mL), and a 6 N aqueous HCl solution (89 µL, 0. 53 mmol) was added to the solution at 0°C. The mixture was evaporated to dryness under vacuum, and the residue was purified by crystallization from MeOH/ether to afford the desired product(137 mg, 95%) as white crystals. The product was dissolved in H₂O (20 mL), filtered through a membrane filter (0. 45 µL), and the filtrate was freeze-dried to afford the titled compound as a white solid.
IR (neat) 3339 (NH), 1755 (C=O), 1680 (C=O) cm⁻¹;
¹H NMR (DMSO-*d*₆) δ 0. 94 (t, *J*= 7. 5 Hz, 3 H, CH₂CH₂C*H*₃), 0. 96 (d, *J*= 7. 2 Hz, 3 H, CHC*H*₃), 0. 99 (d, *J*= 6. 9 Hz, 3 H, CHC*H*₃), 1. 37 (t, *J*= 6. 9 Hz, 3 H, OCH₂C*H*₃), 1. 59-1. 71 (m, 2 H, CH₂C*H*₂CH₃), 2. 14-2. 28 (m, 1 H, C*H*CH₃), 2. 59 (t, *J*= 7. 5 Hz, 2 H, C*H*₂CH₂CH₃), 2. 91-3. 16 (m, 2 H, 2 SO₂NCHₐₓ), 3. 04-3. 92 (m, 9 H, 2 SO₂NCH_{eq}, NC*H*₂CH₂O, 2 H⁺NC*H*ₐₓ, 2 H⁺NC*H*_{eq}, COCH), 3. 99 (s, 3 H, NCH₃), 4. 25 (q, *J*= 6. 9 Hz, 2 H, OC*H*₂CH₃), 4. 42-4. 61 (m, 2 H, NCH₂C*H*₂O), 7. 22 (s, 1 H, H-2), 7. 41 (d, *J*= 9. 0 Hz, 1 H, H-3'), 7. 87 (dd, *J*= 9. 0 Hz, 2. 7 Hz, 1 H, H-4'), 8. 03 (d, *J*= 2. 7 Hz, 1 H, H-6'), 8. 67 (br s, 2 H, 2 H⁺);
MS (FAB) *m*/*z* 604 (MH⁺-2 HCl).

### Example 22

### 2-{5-[4-(3-(N-(tert-Butoxycarbonyl)-L-valinyl)oxypropyl)piperazin-1-ylsulfonyl]-2-ethoxyphenyl}-5-methyl-7-n-propyl-3,5-dihydro-4H-pyrrolo[3,2-d]pyrimidin-4-one

The titled compound was prepared as described in Example 20 by using 2-{2-ethoxy-5-[4-(3-hydroxypropyl)piperazin-1-ylsulfonyl]phenyl}-5-methyl-7-*n*-propyl-3,5-dihydro-4*H*-pyrrolo[3,2-*d*]pyrimidin-4-one in place of 2-{2-ethoxy-5-[4-(2-hydroxyethyl)piperazin-1-ylsulfonyl]phenyl}-5-methyl-7-*n*-propyl-3,5-dihydro-4*H*-pyrrolo[3,2-*d*]pyrimidin-4-one.
yield: 80%
mp 132-134 °C (EtOAc/ether/hexanes);
IR (neat) 3329 (NH), 1749 (C=O), 1681 (C=O) cm⁻¹;
¹H NMR (CDCl₃/TMS) δ 0. 85 (d, *J*= 6. 6 Hz, 3 H, CHC*H*₃), 0. 92 (d, *J*= 6. 9 Hz, 3 H, CHC*H*₃), 0. 99 (t, *J*= 7. 5 Hz, 3 H, CH₂CH₂C*H*₃), 1. 42 (s, 9 H, 3 CH₃), 1. 64 (t, *J*= 6. 9 Hz, 3 H, OCH₂C*H*₃), 1. 69-1. 82 (m, 4 H, CH₂C*H*₂CH₃ and CH₂C*H*₂CH₂O), 2. 01-2. 14 (m, 1 H, C*H*CH₃), 2. 41 (t, *J*= 6. 6 Hz, 2 H, C*H*₂CH₂CH₂O), 2. 52 (m, 4 H, 2 NCH₂), 2. 71 (t, *J* = 7. 5 Hz, 2 H, C*H*₂CH₂CH₃), 3. 08 (m, 4 H, 2 SO₂NCH₂), 4. 08 (s, 3 H, NCH₃), 4. 11 (t, *J* = 6. 6 Hz, 2 H, CH₂CH₂C*H*₂O), 4. 10-4. 20 (m, 1 H, COCH), 4. 36 (q, *J*= 6. 9 Hz, 2 H, OC*H*₂CH₃), 4. 95 (br d, *J*= 9. 0 Hz, 1 H, NHBoc), 6. 88 (s, 1 H, H-2), 7. 13 (d, *J* = 8. 7 Hz, 1 H, H-3'), 7. 80 (dd, *J*= 8. 7 Hz, 2. 7 Hz, 1 H, H-4'), 8. 87 (d, *J*= 2. 7 Hz, 1 H, H-6'), 10. 61 (br s, 1 H, NH);
MS (FAB) *m*/*z* 717 (MH⁺).

### Example 23

### 2-{2-Ethoxy-5-[4-(3-(L-valinyl)oxypropyl)piperazin-1-ylsulfonyl]phenyl}-5-methyl-7-n-propyl-3,5-dihydro-4H-pyrrolo[3,2-d]pyrimidin-4-one Dihydrochloride

The titled compound was prepared as described in Example 21 by using 2-{5-[4-(3-(*N*-(*tert*-Butoxycarbonyl)-L-valinyl)oxypropyl)piperazin-1-ylsulfonyl]-2-ethoxyphenyl}-5-methyl-7-*n*-propyl-3,5-dihydro-4*H*-pyrrolo[3,2-*d*]pyrimidin-4-one in place of 2-{5-[4-(2-(*N*-(*tert*-Butoxycarbonyl)-L-valinyl)oxyethyl)piperazin-1-ylsulfonyl]-2-ethoxyphenyl}-5-methyl-7-*n*-propyl-3,5-dihydro-4*H*-pyrrolo[3,2-*d*]pyrimidin-4-one.
yield: 98%
IR (neat) 3332 (NH), 1739 (C=O), 1678 (C=O) cm⁻¹;
¹H NMR (DMSO-*d*₆) δ 0. 93 (t, *J*= 7. 5 Hz, 3 H, CH₂CH₂C*H*₃), 0. 95 (d, *J*= 7. 2 Hz, 3 H, CHC*H*₃), 0. 98 (d, *J*= 6. 6 Hz, 3 H, CHC*H*₃), 1. 36 (t, *J*= 6. 9 Hz, 3 H, OCH₂C*H*₃), 1. 57-1. 70 (m, 2 H, CH₂C*H*₂CH₃), 2. 01-2. 25 (m, 3 H, CH₂C*H*₂CH₂O and C*H*CH₃), 2. 59 (t, *J* = 7. 5 Hz, 2 H, C*H*₂CH₂CH₃), 2. 82-2. 96 (m, 2 H, 2 SO₂NCHₐₓ), 3. 10-3. 32 (m, 4 H, 2 SO₂NCH_{eq} and C*H*₂CH₂CH₂O), 3. 50-3. 62 (m, 2 H, 2 H⁺NC*H*ₐₓ), 3. 76-3. 88 (m, 3 H, 2 H⁺NC*H*_{eq} and COCH), 3. 99 (s, 3 H, NCH₃), 4. 23 (t, *J* = 7. 2 Hz, 2 H, CH₂CH₂C*H*₂O), 4. 24 (q, *J*= 6. 9 Hz, 2 H, OC*H*₂CH₃), 7. 26 (s, 1 H, H-2), 7. 42 (d, *J*= 9. 0 Hz, 1 H, H-3'), 7. 88 (dd, *J*= 9. 0 Hz, 2. 7 Hz, 1 H, H-4'), 8. 00 (d, *J*= 2. 7 Hz, 1 H, H-6'), 8. 67 (br s, 2 H, 2 H⁺);
MS (FAB) *m*/*z* 617 (MH⁺-2 HCl).

### Example 24

### 5-Ethyl-2-{5-[4-(2-phenylacetoxyethyl)piperazin-1-ylsulfonyl]-2-n-propoxyphenyl}-7-n-propyl-3,5-dihydro-4H-pyrrolo[3,2-d]pyrimidin-4-one

The titled compound was prepared as described in Example 20 by using 5-ethyl-2-{5-[4-(2-hydroxyethyl)piperazin-1-ylsulfonyl]-2-*n*-propoxyphenyl}-7-*n*-propyl-3,5-dihydro-4*H*-pyrrolo[3,2-*d*]pyrimidin-4-one and phenylacetic acid in place of 2-{2-ethoxy-5-[4-(2-hydroxyethyl)piperazin-1-ylsulfonyl]phenyl}-5-methyl-7-*n*-propyl-3,5-dihydro-4*H*-pyrrolo[3,2-*d*]pyrimidin-4-one and *N*-(*tert*-butoxycarbonyl)-L-valine.
yield: 96%
IR (neat) 3331 (NH), 1735 (C=O), 1665 (C=O) cm⁻¹;
¹H NMR (CDCl₃/TMS) δ 1. 00 (t, *J*= 7. 2 Hz, 3 H, CH₂CH₂C*H*₃), 1. 20 (t, *J* = 7. 5 Hz, 3 H, OCH₂CH₂C*H*₃), 1. 48 (t, *J* = 7. 2 Hz, 3 H, NCH₂C*H*₃), 1. 69-1. 81 (m, 2 H, CH₂C*H*₂CH₃), 1. 99-2. 11 (m, 2 H, OCH₂C*H*₂CH₃), 2. 51 (m, 4 H, 2 NCH₂), 2. 58 (t, *J* = 5. 7 Hz, 2 H, NC*H*₂CH₂O), 2. 73 (t, *J*= 7. 5 Hz, 2 H, C*H*₂CH₂CH₃), 3. 02 (m, 4 H, 2 SO₂NCH₂), 3. 57 (s, 2 H, O₂CCH₂), 4. 14 (t, *J*= 5. 7 Hz, 2 H, NCH₂C*H*₂O), 4. 25 (t, *J*= 6. 6 Hz, 2 H, OC*H*₂CH₂CH₃), 4. 46 (q, *J*= 7. 2 Hz, 2 H, NC*H*₂CH₃), 6. 97 (s, 1 H, H-2), 7. 14 (d, *J*= 8. 7 Hz, 1 H, H-3'), 7. 17-7. 25 (m, 5 H, 5 Ph-H), 7. 80 (dd, *J*= 8. 7 Hz, 2. 7 Hz, 1 H, H-4'), 8. 90 (d, *J*= 2. 7 Hz, 1 H, H-6'), 10. 71 (br s, 1 H, NH);
MS (FAB) *m*/*z* 650 (MH⁺).

### Example 25

Production of tablets (Direct compression)

| | mg/tablet |
|---|---|
| Active ingredient | 5. 0 |
| Lactose | 14. 1 |
| Crospovidone USNF | 0. 8 |
| Magnesium Stearate | 0. 1 |
| Total weight | 20 mg |

The active ingredient was sieved and blended with the excipients. The resultant mix was compressed into tablets.

Alternatively, the active ingredient and lactose were dissolved in water and freeze-dried. Then, the dried mixture was blended with the excipients and was compressed into tablets.

### Example 26

Production of tablets (Wet granulation)

| | mg/tablet |
|---|---|
| Active ingredient | 5. 0 |
| Polysorbate 80 | 0. 3 |
| Lactose | 16. 0 |
| Starch | 4. 0 |
| Colloidal Silicon Dioxide | 2. 7 |
| Magnesium Stearate | 2. 0 |
| Total weight | 30 mg |

The active ingredient was sieved and blended with the lactose and starch. The polysorbate 80 was dissolved in purified water. Suitable volumes of the polysorbate 80 solution were added and the powders were granulated. After drying, the granules were screened and blended with the colloidal silicon dioxide and magnesium stearate. The granules were then compressed into tablets.

### Example 27

Production of powder and encapsulated medicine

| | mg/capsule |
|---|---|
| Active ingredient | 5. 0 |
| Lactose | 14. 8 |
| Polyvinyl pyrrolidone | 10. 0 |
| Magnesium Stearate | 0. 2 |
| Total weight | 30 mg |

The active ingredient was sieved and blended with the excipients. The mix was filled into No. 5 hard gelatin capsules using suitable equipment.

### Example 28: Biological activity

The rabbit platelet PDE V was prepared using the method described by Hidaka et al. (*Biochim. Biophys. Acta*., 429: 485 - 497, 1976) with minor modification. The plateletrich plasma (PRP) was prepared by centrifugation of freshly obtained heparinized whole blood at 360 g. Platelets were isolated from the PRP by centrifugation at 1,200 g. Platelet homogenates were prepared in the homogenization buffer (50 mM Tris-HCl buffer containing 1 mM MgCl₂, pH 7.4) by sonication for 30 s per 1 mL. The homogenized solutions were then centrifuged at 40,000 × g for 2 h at 4 °C. The supernatant was loaded on the DEAE-cellulose column (20 mL bed volume, Sigma) pre-equilibrated with equilibration buffer (50 mM Tris-acetate containing 3.75 mM 2-mercaptoethanol, pH 6.0). The column was then washed with 60 mL of equilibration buffer. The PDE isozymes were eluted using a continuous gradient of 0 to 600 mM sodium acetate in the equilibration buffer (60 mL total volume). The 1.0 mL fractions were collected. A flow rate of 60 mL/h was used throughout the ion-exchange chromatography procedure. PDE activities on cAMP and cGMP were characterized as described below. The characterized fractions were pooled and stored at -80 °C until the inhibition studies.

The cyclic nucleotide PDE V activity was determined using PDE SPA assay kit (Amersham Pharmacia biotech, UK). Each reaction mixture (100 µL total volume) consisted of the column elute containing PDE V (10 µL), [³H]-cGMP (5 µCi/mL), bovine serum albumin (0. 5 mg/mL), and MgCl₂ (5 mM) in Tris-HCl buffer (15 mM, pH 7. 5). The reactions were initiated by the addition of PDE V and the samples were incubated at 30 °C for 30 min, after which the reaction was stopped by the addition of 50 µL of SPA beads. The reaction tube was then settled for 20 min and was counted on the scintillation counter (Tri-carb 1500, Packard, USA). For the inhibition study of PDE V activity, the test compounds were dissolved in dimethyl sulfoxide (DMSO) and was diluted with distilled water. The final concentration of DMSO was less than 0. 2% (v/v). All the inhibition experiments were conducted under the conditions where the level of cGMP hydrolysis did not exceed 15%, and the product formation increased linearly with time and the amount of enzyme.

The following Table illustrates the in vitro activities for a range of the compounds of the invention as inhibitors of cGMP PDE V.

**TABLE**

| EXAMPLE NO. | IC₅₀ (nM) |
|---|---|
| 7 | 2.84 |
| 10 | 5.73 |
| 12 | 8.87 |
| 14 | 8.80 |
| 16 | 14.4 |
| 17 | 7.65 |
| 19 | 1.48 |
| 24 | 8.68 |

### Example 29: Safety Profile

Several compounds of the invention have been tested at doses of up to 10 mg/kg p. o. in rats with no untoward effects being observed, and up to 100 mg/kg p. o. in rats with no death being observed.

## Claims

1. A compound of the formula (1) and pharmaceutically acceptable salts and solvates (e. g. hydrates) thereof, 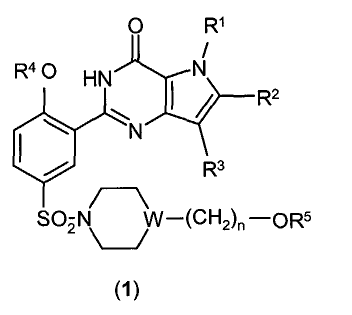 wherein
R¹ is H; or C₁-C₃ alkyl optionally substituted with one or more fluoro atoms;
R² is H; a halogen atom; C₁-C₆ alkyl optionally substituted with OH, or one or more fluoro atoms;
R³ is H; C₁-C₆ alkyl optionally substituted with OH, C₁-C₃ alkoxy, or one or more fluoro atoms; C₃-C₆ cycloalkyl; C₂-C₆ alkenyl; or C₂-C₆ alkynyl;
R⁴ is C₁-C₆ alkyl optionally substituted with one or more fluoro atoms; C₂-C₆ alkenyl; C₂-C₆ alkynyl; or C₃-C₆ cycloalkyl;
W is N; or CH;
n is an integer of from 1 to 6;
R⁵ is COR⁶; COOR⁶; or COCH(R⁷)NH₂;
R⁶ is phenyl or aromatic heterocyclic group wherein said group is optionally substituted with 1 to 4 substituents selected from the group consisting of halogen atom, C₁-C₄ alkyl, C₁-C₄ alkoxy, OH, NH₂, NO₂, CN, COOH, C₁-C₄ aminoalkyl, C₁-C₄ alkylamino, C₁-C₄ acyl, C₁-C₄ acylamino, C₁-C₄ alkylthio, C₁-C₄ perfluoroalkyl, C₁-C₄ perfluoroalkoxy and C₁-C₄ alkoxycarbonyl; C₁-C₁₆ alkyl, C₂-C₁₆ alkenyl or C₂-C₁₆ alkynyl wherein said group is optionally substituted with one or more halogen atoms, C₁-C₄ alkoxy, OH, NH₂, NO₂, CN, COOH, C₁-C₄ aminoalkyl, C₁-C₄ alkylamino, C₁-C₄ acyl, C₁-C₄ acylamino, C₁-C₄ alkylthio, C₁-C₄ perfluoroalkyl, C₁-C₄ perfluoroalkoxy, C₁-C₄ alkoxycarbonyl or with phenyl or aromatic heterocyclic group wherein said group is optionally substituted with 1 to 4 substituents selected from the group consisting of halogen atom, C₁-C₄ alkyl, C₁-C₄ alkoxy, OH, NH₂, NO₂, CN, COOH, C₁-C₄ aminoalkyl, C₁-C₄ alkylamino, C₁-C₄ acyl, C₁-C₄ acylamino, C₁-C₄ alkylthio, C₁-C₄ perfluoroalkyl, C₁-C₄ perfluoroalkoxy and C₁-C₄ alkoxycarbonyl;
R⁷ represents the residue of any naturally occurring amino acid.

2. The compound according to Claim 1, wherein
R¹ is H; methyl; or ethyl;
R² is H; methyl; or a halogen atom;
R³ is C₁-C₄ alkyl optionally substituted with one or more fluoro atoms;
R⁴ is ethyl; *n*-propyl; or allyl;
W is N;
n is an integer of from 1 to 6;
R⁵ is COR⁶; or COOR⁶;
R⁶ is C₁-C₄ alkyl.

3. The compound according to Claim 2, wherein
R¹ is methyl; or ethyl;
R² is H;
R³ is ethyl; 2-fluoroethyl; *n*-propyl; or 3-fluoropropyl;
R⁴ is ethyl; or *n*-propyl;
W is N;
n is an integer of from 2 to 4;
R⁵ is COR⁶; or COOR⁶;
R⁶ is C₁-C₄ alkyl.

4. The compound according to Claim 3, wherein said compound is selected from the group consisting of:
2-{5-[4-(2-Acetoxyethyl)piperazin-1-ylsulfonyl]-2-*n*-propoxyphenyl}-5-ethyl-7-*n*-propyl-3,5-dihydro-4*H*-pyrrolo[3,2-*d*]pyrimidin-4-one;
5-Ethyl-2-{5-[4-(2-propionyloxyethyl)piperazin-1-ylsulfonyl]-2-*n*-propoxyphenyl}-7-*n*-propyl-3,5-dihydro-4*H*-pyrrolo[3,2-*d*]pyrimidin-4-one;
2-{5-[4-(2-Butyryloxyethyl)piperazin-1-ylsulfonyl]-2-*n*-propoxyphenyl}-5-ethyl-7-*n*-propyl-3,5-dihydro-4*H*-pyrrolo[3,2-*d*]pyrimidin-4-one;
5-Ethyl-2-{5-[4-(2-isobutyryloxyethyl)piperazin-1-ylsulfonyl]-2-*n*-propoxyphenyl}-7-*n*-propyl-3,5-dihydro-4*H*-pyrrolo[3,2-*d*]pyrimidin-4-one;
5-Ethyl-2-{5-[4-(2-methoxycarbonyloxyethyl)piperazin-1-ylsulfonyl]-2-*n*-propoxyphenyl}-7-*n*-propyl-3,5-dihydro-4*H*-pyrrolo[3,2-*d*]pyrimidin-4-one;
2-{5-[4-(2-Ethoxycarbonyloxyethyl)piperazin-1-ylsulfonyl]-2-*n*-propoxyphenyl}-5-ethyl-7-*n*-propyl-3,5-dihydro-4*H*-pyrrolo[3,2-*d*]pyrimidin-4-one;
5-Ethyl-2-{5-[4-(2-*n*-propoxycarbonyloxyethyl)piperazin-1-ylsulfonyl]-2-*n*-propoxyphenyl}-7-*n*-propyl-3,5-dihydro-4*H*-pyrrolo[3,2-*d*]pyrimidin-4-one;
5-Ethyl-2-{5-[4-(2-isopropoxycarbonyloxyethyl)piperazin-1-ylsulfonyl]-2-*n* propoxyphenyl}-7-*n*-propyl-3,5-dihydro-4*H*-pyrrolo[3,2-*d*]pyrimidin-4-one;
2-{5-[4-(2-*n*-Butoxycarbonyloxyethyl)piperazin-1-ylsulfonyl]-2-*n*-propoxyphenyl}-5-ethyl-7-*n*-propyl-3,5-dihydro-4*H*-pyrrolo[3,2-*d*]pyrimidin-4-one;
2-{5-[4-(2-Acetoxyethyl)piperazin-1-ylsulfonyl]-2-*n*-propoxyphenyl}-5-ethyl-7-(3-fluoropropyl)-3,5-dihydro-4*H*-pyrrolo[3,2-*d*]pyrimidin-4-one;
5-Ethyl-7-(3-fluoropropyl)-2-{5-[4-(2-propionyloxyethyl)piperazin-1-ylsulfonyl]-2-*n*-propoxyphenyl}-3,5-dihydro-4*H*-pyrrolo[3,2-*d*]pyrimidin-4-one;
2-{5-[4-(2-Butyryloxyethyl)piperazin-1-ylsulfonyl]-2-*n*-propoxyphenyl}-5-ethyl-7-(3-fluoropropyl)-3,5-dihydro-4*H*-pyrrolo[3,2-*d*]pyrimidin-4-one;
5-Ethyl-7-(3-fluoropropyl)-2-{5-[4-(2-isobutyryloxyethyl)piperazin-1-ylsulfonyl]-2-*n*-propoxyphenyl}-3,5-dihydro-4*H*-pyrrolo[3,2-*d*]pyrimidin-4-one;
5-Ethyl-7-(3-fluoropropyl)-2-{5-[4-(2-methoxycarbonyloxyethyl)piperazin-1-ylsulfonyl]-2-*n*-propoxyphenyl}-3,5-dihydro-4*H*-pyrrolo[3,2-*d*]pyrimidin-4-one;
2-{5-[4-(2-Ethoxycarbonyloxyethyl)piperazin-1-ylsulfonyl]-2-*n*-propoxyphenyl}-5-ethyl-7-(3-fluoropropyl)-3,5-dihydro-4*H*-pyrrolo[3,2-*d*]pyrimidin-4-one;
5-Ethyl-7-(3-fluoropropyl)-2-{5-[4-(2-*n*-propoxycarbonyloxyethyl)piperazin-1-ylsulfonyl]-2-*n*-propoxyphenyl}-3,5-dihydro-4*H*-pyrrolo[3,2-*d*]pyrimidin-4-one;
5-Ethyl-7-(3-fluoropropyl)-2-{5-[4-(2-isopropoxycarbonyloxyethyl)piperazin-1-ylsulfonyl]-2-*n*-propoxyphenyl}-3,5-dihydro-4*H*-pyrrolo[3,2-*d*]pyrimidin-4-one;
2-{5-[4-(2-*n*-Butoxycarbonyloxyethyl)piperazin-1-ylsulfonyl]-2-*n*-propoxyphenyl}-5-ethyl-7-(3-fluoropropyl)-3,5-dihydro-4*H*-pyrrolo[3,2-*d*]pyrimidin-4-one;
and physiologically acceptable salts and solvates (e. g. hydrates) thereof.

5. A pharmaceutical composition comprising a compound of the formula **(1)** or a pharmaceutically acceptable salt, or a solvate thereof according to any one of Claims 1 to 4, together with a pharmaceutically acceptable diluent or carrier therefor.

6. A compound of the formula **(1)** or a pharmaceutically acceptable salt, or a solvate thereof, or a pharmaceutical composition containing either entity according to any one of Claims 1 to 5, for use in the treatment of impotence, sexual dysfunction in female, stable, unstable and variant (Prinzmetal) angina, hypertension, pulmonary hypertension, congestive heart failure, renal failure, atherosclerosis, conditions of reduced blood vessel patency (e. g. post-percutaneous transluminal coronary angioplasty), peripheral vascular disease, vascular disorders such as Raynaud's disease, inflammatory diseases, stroke, bronchitis, chronic asthma, allergic asthma, allergic rhinitis, glaucoma and diseases **characterized by** disorders of gut motility (e. g. irritable bowel syndrome).

7. The use of a compound of the formula **(1)** or a pharmaceutically acceptable salt, or a solvate thereof, or a pharmaceutical composition containing either entity according to any one of Claims 1 to 5, for the manufacture of a medicament for the treatment of impotence, sexual dysfunction in female, stable, unstable and variant (Prinzmetal) angina, hypertension, pulmonary hypertension, congestive heart failure, renal failure, atherosclerosis, conditions of reduced blood vessel patency (e. g. post-percutaneous transluminal coronary angioplasty), peripheral vascular disease, vascular disorders such as Raynaud's disease, inflammatory diseases, stroke, bronchitis, chronic asthma, allergic asthma, allergic rhinitis, glaucoma and diseases **characterized by** disorders of gut motility (e. g. irritable bowel syndrome).

8. A method of treating or preventing impotence, sexual dysfunction in female, stable, unstable and variant (Prinzmetal) angina, hypertension, pulmonary hypertension, congestive heart failure, renal failure, atherosclerosis, conditions of reduced blood vessel patency (e. g. post-percutaneous transluminal coronary angioplasty), peripheral vascular disease, vascular disorders such as Raynaud's disease, inflammatory diseases, stroke, bronchitis, chronic asthma, allergic asthma, allergic rhinitis, glaucoma and diseases **characterized by** disorders of gut motility (e. g. irritable bowel syndrome), in a mammal (including a human being), which comprises administering to said mammal a therapeutically effective amount of a compound of formula **(1)**, or a pharmaceutically acceptable salt, or a solvate thereof, or a pharmaceutical composition containing either entity, according to any one of Claims 1 to 5.

9. A compound of the formula **(8)**: 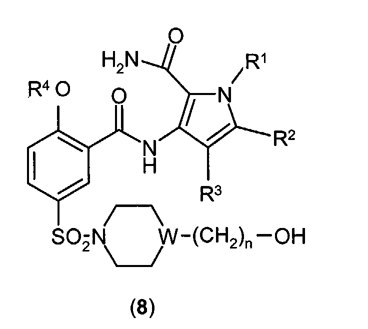 wherein R¹, R², R³, R⁴, W and n are as previously defined in Claim 1.

10. Processes for preparing a compound of the formula **(1)**: 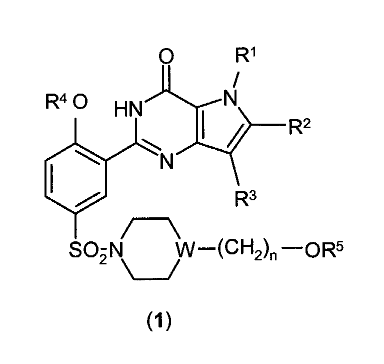 wherein R¹, R², R³, R⁴, W, n and R⁵ are as previously defined in Claim 1, and pharmaceutically acceptable salts thereof, which comprises reacting a compound of the 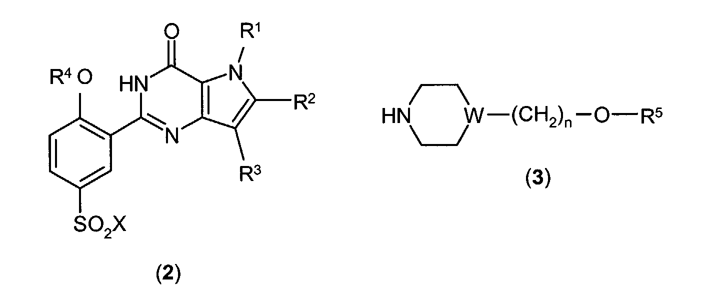 formula **(2)**:
wherein R¹, R², R³ and R⁴ are as previously defined in Claim 1, and X represents a halogen atom, with an appropriate compound of the formula **(3)** under an appropriate reaction condition, wherein W, n and R⁵ are as previously defined in Claim 1.

11. Processes for preparing a compound of the formula **(1)**: 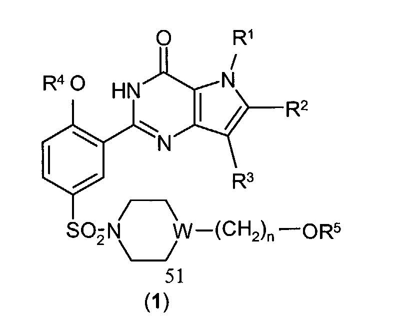 wherein R¹, R², R³, R⁴, W, n and R⁵ are as previously defined in Claim 1, and pharmaceutically acceptable salts thereof, which comprises reacting a compound of the formula **(4)**: 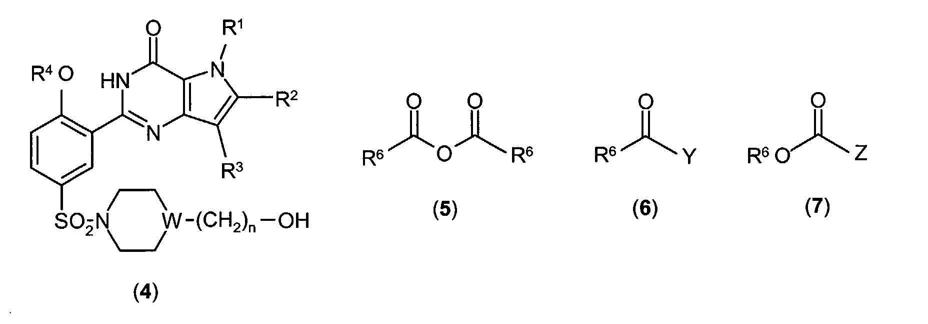 wherein R¹, R², R³, R⁴, W, and n are as previously defined in Claim 1, with an appropriate compound of the formula (**5**), (**6**) or (**7**) under an appropriate reaction condition: wherein R⁶ is as previously defined in Claim 1, and Y is OH or a halogen atom, and Z is a halogen atom or a phenoxy group optionally substituted with one or more NO₂ groups.
